# EUROPEAN PATENT APPLICATION

(11) **EP 4 109 101 A1**
(43) Date of publication of application: **28.12.2022**
(21) Application number: 21181465.2
(22) Date of filing: 24.06.2021
(51) Int. Cl.: G01N 33/569

(54) **PORTABLE TESTING DEVICE FOR TESTING SUSCEPTIBILITY OF A BIOLOGICAL PROBE TO ONE OR MORE BIOACTIVE SUBSTANCES**

(71) Applicant: Technische Universität München, 80333 München (DE)
(72) Inventor: Hayden, Oliver, 85368 Moosburg (DE); Wali, Sarah, 81373 München (DE); Alarcón-Mostajo, Maurice-Sebastian, 80637 München (DE); Sabersky-Müssigbrodt, Henning, 82041 Oberhaching (DE)
(74) Representative: Lucke, Andreas

(57) **Abstract**

The invention refers to a testing device (10) for testing the susceptibility of a biological probe to one or more bioactive substances. The testing device comprises a liquid-impermeable first substrate (12), a second substrate (14) arranged or arrangeable over the first substrate (12), wherein the second substrate (14) is liquid-permeable and liquid-impregnable, and a third substrate (16) arranged or arrangeable over the second substrate (14), wherein the third substrate (16) is liquid-impermeable. The first substrate and/or the third substrate are configured for encapsulating the second substrate. At least a portion of the second substrate (14) is exposable to an environment of the testing device for receiving the biological probe. The invention further refers to a testing kit comprising such a testing device and at least one of one or more bioactive substances, a biological culture medium, and a bacterial marking substance configured for undergoing a property change in the presence of bacteria. The invention further refers to a corresponding method of testing the susceptibility of a biological probe to one or more bioactive substances.

## Description

### FIELD OF THE INVENTION

The present invention is in the field of medical technology and refers in particular to a testing device for testing the susceptibility of a biological probe to one or more bioactive substances.

### BACKGROUND OF THE INVENTION

Increasing rates of antimicrobial resistance to commonly used drugs are being observed in a number of bacterial diseases and infections. Some types of infectious bacteria are even developing antimicrobial resistance to a plurality of typically employed drugs. Antimicrobial resistance is increasing rapidly in particular in developing countries due to an extensive use of antibiotic drugs under reduced medical supervision or even none at all.

The rapid spread in antimicrobial resistances poses a challenge to healthcare systems, inasmuch as it increases the need of patient-specific treatment requiring preliminary testing of antimicrobial resistance for a number of drugs, particularly antibiotics, in order to identify potentially effective drug-based treatments and to discard non-effective treatments. The lack of such preliminary testing can imply the risk of improper treatment, including incorrect dosage, incorrect drug combinations, and useless or even counterproductive treatment.

The logistics of such preliminary testing, which usually involves extraction and incubation of biological probes obtained from a patient in central testing facilities, are however complex and require corresponding networks of health and laboratory professionals, health expenditure control supervision, as well as proper infrastructure for probe collection and transportation. These limitations are particularly severe in rural and remote areas as well as in third world countries lacking good infrastructural networks.

The incubation process is used for observing the biological evolution of bacteria under the influence of different drugs so as to determine the corresponding susceptibilities of bacteria. It is generally performed on agar plates using small discs containing different antibiotics or papers impregnated with antibiotics, which are dropped in different zones of the bacteria culture on an agar plate, which is a nutrient-rich environment in which bacteria can grow. The presence of the agar fosters bacteria growth. The antibiotic diffuses into the area surrounding each disc or paper and a region of impaired bacterial growth becomes visible in such area. The test results are usually referred to as "antibiogram". Since the concentration of the antibiotic is highest at the centre of the corresponding disc or paper and lowest at the edges thereof, the diameter of the corresponding region of impaired bacterial growth is representative of the so-called "minimum inhibitory concentration" required for inhibiting bacterial growth.

Not only incubation makes testing at highly specialised laboratory facilities necessary. Since testing is performed with infectious substances, only staff with proper laboratory training is usually allowed to manipulate such probes for testing using most of the testing devices known from the prior art.

The aforesaid limitations further imply a significant time lapse between probe collection and test result delivery. Even in highly developed countries having proper infrastructures, antibiotic susceptibility diagnostics, for example for urinary tract infection (UTI), typically requires at least 24h to 48h from probe collection until test results can be reported to a healthcare professional, who must then contact the patient for communicating the test results and eventually initiating proper treatment. The real time lapse between probe collection and test result availability might however increase depending on the working days and availability of testing and healthcare facilities. This typically requires initiating first a non-optimal preliminary treatment, which must then be corrected or adapted once the test results are available.

Existing molecular diagnostic tests using point-of-care-tests (POCT) are costly solutions requiring a sophisticated environment and professional expertise for usage. However, these are usually not able to report antimicrobial resistances, which is the reason why centralized testing in dedicated laboratories continues to be the gold standard. More recent developments on polymerase chain reaction (PCR) and sequencing testing try to circumvent some of these limitations but are even more costly and hence provide no solution accessible to resource-limited environments.

There is hence room for technical improvement in the field of susceptibility testing of biological probes.

### SUMMARY OF THE INVENTION

The present invention aims at overcoming the aforementioned disadvantages of the prior art, in particular by providing a testing device for testing the susceptibility of a biological probe to one or more bioactive substances, which requires no expert use, can be used as POCT without requiring transportation to a central testing laboratory and can be easily manufactured at reduced costs. A "bioactive substance" may refer herein in particular to a drug for human or animal use, but may also refer to any other biocidal substance. The aforesaid aim of the invention is achieved by a testing device for testing the susceptibility of a biological probe to one or more bioactive substances according to claim 1, by a testing kit according to claim 13 and by a corresponding method according to claim 15. Preferred embodiments of the invention are defined in the appended dependent claims.

The testing device of the invention, which may in particular be a portable testing device, comprises a plurality of substrates. The plurality of substrates comprises a first substrate, a second substrate arranged or arrangeable above the first substrate, and a third substrate arranged or arrangeable above the second substrate. The second substrate may be arranged or arrangeable directly on the first substrate and, additionally or alternatively, the third substrate may be arranged or arrangeable directly on the second substrate, although in either case, one or more additional substrates may be arranged between the first substrate and the second substrate and/or between the second substrate and a third substrate. Thus, in one of the structurally simplest implementations of the testing device of the invention, the testing device may comprise three substrates that are arranged or arrangeable on top of each other.

The first substrate is liquid-impermeable. "Liquid-impermeable" may refer herein in particular to the fact that a substrate substantially prevents a liquid from flowing therethrough, although said liquid may penetrate partly into said substrate in some cases. A liquid-permeable substrate prevents liquids from flowing therethrough, and is hence effectively also bacteria-impermeable, i.e. prevents bacteria from migrating therethrough.

The first substrate may optionally further be "gas-permeable", in particular oxygen-permeable and/or humidity-permeable. Thus, a substrate being "gas-permeable" may refer herein to the fact that a gas, in particular oxygen and/or humidity (i.e. water vapour), may pass through said substrate. In other words, the first substrate may be configured to prevent liquids and bacteria from passing therethrough while optionally allowing gases, for example oxygen and/or humidity (water vapor), to pass therethrough.

A substrate or layer of a liquid-impermeable material can be used for trapping a liquid, in particular a liquid comprising the biological probe. For example, the first substrate may comprise or may be made of a liquid-impermeable material, which may in particular be or comprise a biocompatible material. The first substrate may for example comprise or be of a plastic material such as polyethylene. The liquid-impermeable material of the first substrate may further be a gas-permeable material.

The second substrate is liquid-permeable, and is in particular suitable for being impregnated with a biological culture medium for supporting bacterial growth, such as for example a lysogeny broth (or LB) medium (also called Luria broth medium) for supporting bacterial proliferation. "Liquid-permeable" may refer herein in particular to the ability of the second substrate of allowing the liquid to flow and diffuse through and within the second substrate.

The second substrate is further liquid-impregnable. "Liquid-impregnable" may refer herein in particular to the ability of the second substrate to retain within the second substrate a liquid absorbed into the second substrate. Thus, because the second substrate is "liquid-permeable" and "liquid-impregnable", when a liquid, in particular a liquid comprising a biological probe, such as for example a urine probe, contacts the second substrate, the liquid penetrates into the second substrate and can diffuse therethrough impregnating the second substrate, wherein at least a part of the liquid remains embedded in the second substrate impregnating the second substrate instead of flowing back out of the second substrate. The second substrate may for example comprise or be made of a paper material, for example absorbent paper of the kind used for conventional pH tests or blotting paper.

Notably, the "liquid comprising a biological probe" may refer herein to a biological probe provided in liquid state, for example a urine probe of a patient, or to a combination of a biological probe provided in solid state and a liquid solvent in which the solid-state biological probe may be solved, for example a solid or powder-like probe of a food product solved in water or in any other appropriate liquid.

Therefore, the second substrate can act as a reaction and/or incubation medium, in which the biological probe may react with other substances present in the second substrate or added thereto, for example with the one or more bioactive substances and/or with a bacterial marking substance configured for undergoing a property change in the presence of bacteria.

"Bacterial marking substance" refers to a substance configured for undergoing a property change, i.e. a change in a perceivable property of the bacterial marking substance, in the presence of bacteria, such that, when bacteria grow in contact with the bacterial marking substance, such bacterial growth is signalled by a change of a property of the bacterial marking substance, for example a visual appearance, a smell, a texture or a phase composition.

The bacterial marking substance may in particular be a chromogenic substance configured for undergoing a change in colouration in the presence of bacteria, such that when bacteria grow in the probe receiving portions of the second substrate, such bacterial growth is signalled by a change of colouration of the chromogenic substance, where a colour intensity may be indicative or the concentration of bacteria. The chromogenic substance may for example be or comprise a pH sensitive dye or a nitrite sensitive dye, enzymatic indicators such as X-Gal or 6-chloro-3-indolyy-glucuronide cyclohexylammonium salt.

The third substrate is liquid-impermeable . The third substrate may comprise or be made of a liquid-impermeable material. The third substrate may optionally further be "gas-permeable", in particular oxygen-permeable and/or humidity-permeable. The third substrate may be configured to prevent liquids and bacteria from passing therethrough while optionally allowing gases, for example oxygen and/or humidity (water vapor), to pass therethrough. The first and third substrates may have the same or different material composition. The third substrate may comprise or may be made of a plastic or silicon material, such as for instance PDMS, which is liquid-impermeable and oxygen-permeable.

Since the liquid-permeable and liquid-impregnable second substrate is arranged between the first substrate and the third substrate, which are both liquid-impermeable, the first substrate and/or the third substrate can effectively trap a liquid within the second substrate preventing it from flowing away from the second substrate through the first and/or third substrate, respectively.

In some embodiments, the first substrate and/or the third substrate may optionally further be fluid-impermeable, i.e. both liquid-impermeable and gas-impermeable, in order to prevent both liquids and gases from passing therethrough, such as to prevent a liquid arranged in the second substrate, not only from flowing away, but also from evaporating away.

However, in some embodiments, the first substrate and/or the third substrate may be liquid-impermeable and gas-permeable, in particular oxygen-permeable and/or humidity-permeable, in order to prevent liquids from passing therethrough while allowing gases, in particular oxygen and/or water vapor, to flow therethrough. In any case, since the first and third substrates are liquid-impermeable, they are also impermeable to bacteria, i.e. prevent bacteria from migrating through the respective substrate.

According to the invention, the first substrate and/or the third substrate are configured to encapsulate the second substrate. The first substrate and/or the third substrate may encapsulate the second substrate liquid-tightly and/or with gas permeability, in particular with oxygen permeability and/or with humidity permeability (i.e. permeability to water vapour).

"Encapsulating" may refer herein in particular to the ability of the first substrate and/or third substrate to form a liquid-impermeable structure surrounding the second substrate such as to prevent a fluid impregnated in the second substrate from flowing away from the second substrate. For example, the first substrate may cover and encapsulate a lower surface of the second substrate while the third substrate may cover and encapsulate an upper surface of the second substrate opposite to the lower surface, wherein the first substrate or the third substrate may optionally further cover and encapsulate some or all of the side surfaces of the second substrate extending between the upper surface and the lower surface.

According to the invention, at least a portion of the second substrate is exposable to an environment of the testing device for receiving the biological probe. In other words, the structure of the testing device is such that the at least one portion of the second substrate can be brought into contact and/or fluid communication with a liquid substance, which then diffuses through the second substrate and impregnates the second substrate due to the second substrate being liquid-permeable and liquid-impregnable. The "liquid substance" may in particular correspond to and/or comprise one or more of the biological probe, one of the one or more bioactive substances, a bacterial marking substance and/or a biological culture medium.

Once a liquid substance has access to the at least one exposed portion of the second substrate, the liquid substance impregnates the second substrate and may react therein with any other substance(s) that may be present in the second substrate or that may be subsequently added to the second substrate. Thereby, the encapsulating effect of the first substrate and/or the third substrate ensures that the substances that are contained in the second substrate in order to react with each other stay within the second substrate and cannot escape through the first substrate and/or the third substrate.

For example, the second substrate can be impregnated with a biological culture medium for supporting bacterial proliferation, a bacterial marking substance configured for undergoing a property change in the presence of bacteria, and with a dry sample of one of the one or more bioactive substances, which may for example be an antibiotic drug. In a time lapse between manufacturing and use, the second substrate and the substances impregnated therein may be protected from degradation by the encapsulation provided by the first substrate and/or the third substrate. In order to use the testing device of the invention, the at least one portion of the second substrate can be exposed to the environment of the testing device for receiving the biological probe. "Exposed" may refer herein to the ability of the at least one portion of the second substrate of receiving the biological probe from an environment of the testing device without any other component, in particular any other substrate, being interposed between the probe receiving portions and said environment.

For example, an exposed portion of the second substrate maybe brought in contact with a flow of a liquid comprising the biological probe or with a liquid comprising the biological probe contained in a probe container. The biological probe may then diffuse through the second substrate impregnating the second substrate and thereby unleash a reaction with the remaining substance(s) present in the second substrate. The first substrate and/or the third substrate may then be arranged such that they provide the encapsulation that fluidly isolates the second substrate from an environment of the testing device, such that the reaction between all substances present in the second substrate, i.e. the incubation of the biological probe, can take place protected from environmental disruptions.

The testing device of the invention can be used for testing the susceptibility of a biological probe received in the second substrate, such as a urine probe or any other probe provided in liquid state or provided in solid state and solved in a liquid solvent, for example of a human or animal urine probe, to each of the one or more bioactive substances that are also received in the second substrate. In particular, the testing device of the invention may be used for testing the susceptibility of one biological probe received in the second substrate to one bioactive substance that is also received in the second substrate, although other uses of the testing device of the invention are also foreseen within the context of the present invention.

When a biological probe is dispensed to the second substrate, which may also comprise a biological culture medium, a bacterial marking substance, and one of the one or more bioactive substances, bacteria present in the biological probe may grow fostered by the biological culture medium of the second substrate, thereby possibly triggering a property change of the bacterial marking substance, depending on a susceptibility of the biological probe to that particular bioactive substance.

If the biological probe comprises bacteria that are susceptible to the bioactive substance present in the second substrate, bacterial growth is impaired in the second substrate. In particular, bacterial growth may be locally impaired in the second substrate to a lesser or greater degree depending on a local concentration of the bioactive substance in the second substrate. As a consequence, there may be either no property change of the bacterial marking substance or a less perceivable property change as compared to other portions of the second substrate that may be impregnated with a smaller concentration of the bioactive substance or as compared to another second substrate that is not impregnated with any bioactive substance and may operate as a negative control. As a result, the bioactive substance impregnating the second substrate can be identified as potentially effective for treating a bacterial infection present in the tested biological probe if no property change of the bacterial marking substance or only a reduced or partial property change of the bacterial marking substance is observed in the second substrate.

If the biological probe contains bacteria that are resistant, i.e. immune, to the bioactive substance present in the second substrate, bacteria will grow in the second substrate despite the presence of the bioactive substance, inasmuch as bacterial growth cannot be impaired by such bioactive substance. Consequently, there will be a property change of the bacterial marking substance, in particular a property change more pronounced than in the previously mentioned cases in which bacteria are susceptible to a bioactive substance. As a result, the bioactive substance impregnating a second substrate of a testing device in which the bacterial marking substance undergoes a property change, or at least undergoes a property change that is more pronounced as compared to other parts of the second substrate impregnated with another bioactive substance or as compared to another second substrate impregnated with another (effective) bioactive substance, may be identified as an ineffective bioactive substance for treating a bacterial infection present in the tested biological probe. If the bioactive substance is for example a drug, such drug can be discarded as an effective treatment option.

The testing device of the invention allows testing the susceptibility of the biological probe to a bioactive substance without requiring any expert use, any specialised training or any intervention of medically trained staff, and possibly even without requiring any further laboratory equipment. The testing device can be operated in a simple and if necessary confidential manner by the patients themselves. This can be advantageous in view of the prejudices some cultures have regarding infectious diseases such as urinary tract infection (UTI). The testing device of the invention can be used as a POCT (point-of-care-test) testing device for obtaining susceptibility results *in situ* without the need of transporting probes to a remote testing facility, thereby considerably reducing logistic requirements and time of response until test results are obtained.

Further, the testing device of the invention can be easily manufactured at reduced costs, for example in a roll-to-roll process, and has reduced environmental impact as compared to the use of microbial cultures in solid media such as Petri dishes and remote incubators.

It is also possible to impregnate one or more of the substrates of the plurality of substrates, in particular a substrate of the plurality of substrates arrangeable next to the second substrate, with an inhibiting substance configured for inhibiting one or more predefined probe components present in a biological probe. For example, the inhibiting substance may be or comprise a chemical inhibitor or a biochemical inhibitor configured for inhibiting enzymes typically present in a urine probe. Thereby, unwanted components of a biological probe to be tested that may possibly affect and/or falsify the results of the susceptibility test can be inhibited before performing a susceptibility test using the testing device of the present invention, thus reducing the risk of false results.

According to preferred embodiments, the second substrate may comprise or be made of a liquid-permeable material. The liquid-permeable material may be a porous material, in particular paper, preferably having a pore size of 0.3 µm or less, more preferably of 0.1 µm or less. The second substrate may hence be gas-permeable, in particular humidity-permeable and/or oxygen permeable, in order to provide proper environmental conditions for bacterial incubation within the testing device.

In some preferred embodiments, the first substrate, which may in particular be configured as a lowermost substrate of the plurality of substrates, may comprise an adhesive material, in particular an adhesive layer, suitable for adhering to testing device to the body, preferably to the skin of a patient, in particular a human or animal patient. The adhesive material may for example comprise or be a biocompatible glue such as a tackified acrylic adhesive or an hypoallergenic polyethylene.

The testing device may preferably further comprise a removable cover for covering the adhesive material or layer, for example a peelable film. The adhesive material or layer may be used for attaching the testing device to a surface, for example to the body of a patient, in particular of the patient from which the biological probe has been obtained, in order to use the patient's own body warmth and humidity for incubating the biological probe. However, other embodiments need not comprise any adhesive material or layer and may be introduced in a standard incubator for incubation.

For example, after dispensing the biological probe to the second substrate and after bringing all substrates of the plurality of substrates containing substances in contact with each other in order to let the biological probe react with the bioactive substance and with the bacterial marking substance, the adhesive material can be used for attaching the testing device to the body of the patient from which the biological probe was obtained, possibly previously removing the removable cover, and the testing device may remain attached to the patient for incubation. The body of the patient may thereby serve as a portable incubator for the bacteria contained in the biological probe eliminating the necessity of professionally-supervised incubation in specialised laboratory facilities. An incubation time less than 24 hours is usually sufficient for obtaining susceptibility results with good reliability.

In some preferred embodiments, the substrates of the plurality of substrate arranged above the second substrate, in particular the third substrate and possibly also a fourth substrate and/or a fifth substrate to be described in further detail below, are at least partly transparent and/or are made of a transparent material at least in part, such as to allow an optical access to the second substrate from an exterior of the testing device. Thus, the testing device of the invention, in particular the substrates that are arranged above the second substrate, may be partly or totally transparent for optically connecting the second substrate with an exterior of the testing device. It may be hence possible to optically observe the second substrate, and in particular a property change of the bacterial marking substance therein, for example a change in colouration of a chromogenic substance, from an exterior of the testing device.

According to some preferred embodiments, at least some of the substrates of the plurality of substrates, possibly all of them, may be formed as respective substrate layers. The testing device may hence be configured as a layer-based device comprising a stack of layers arranged or arrangeable one upon the other, each layer corresponding to one or more of the substrates of the plurality of substrates. Such a device may be easily manufactured at reduced costs and can be easily transported and stored. Each of the substrates/layers may have a thickness from 100 µm to 2000 µm, preferably from 180 µm to 1500 µm, more preferably from 500 µm to 1500 µm. An overall thickness of the testing device may hence be from 4000 µm to 10000 µm, preferably from 4000 µm to 7000, more preferably from 4000 µm to 5000 µm.

Additionally or alternatively, at least some of the substrates of the plurality of substrates, possibly all of them, may have a strip-like shape. Thus, the testing device of the invention as a whole may have a strip-like shape.

In preferred embodiments, the second substrate may be arrangeable in a probe collection arrangement and in an incubation arrangement. In the probe collection arrangement, the at least one portion of the second substrate may be exposed to an environment of the testing device for receiving the biological probe. In the incubation arrangement, the second substrate may be protected from said environment of the testing device, in particular by the remaining substrates of the plurality of substrates. Thus, the second substrate may be arranged in the probe collection arrangement for exposing the at least one portion of the second substrate for receiving the biological probe, be it directly into the second substrate or indirectly through another one of the substrates (or an exposable partial substrate of the second substrate). Once the second substrate contains the biological probe, the second substrate may be arranged in the incubation arrangement protected from the environment, for example by the surrounding first substrate, third substrate and possibly by any other present substrate. In the incubation arrangement, the biological probe in the second substrate may react with the remaining substance(s) impregnated in the second substrate and/or in the substrates adjacent to the second substrate.

In preferred embodiments of the invention, at least some of the substrates of the plurality of substrates may be mutually attached or attachable by respective mutually overlapping attachment portions. Such attachment portions may preferably correspond to respective end portions of the substrates of the plurality of substrates. In an open configuration of the plurality of substrates, at least some of the substrates of the plurality of substrates may be spread apart from each other, such that the at least one portion of the second substrate may be exposed to an environment of the testing device for receiving the biological probe. In a closed configuration of the plurality of substrates, the substrates of the plurality of substrates may completely overlap each other. The open configuration may correspond to the aforementioned probe collection arrangement and the closed configuration may correspond to the aforementioned incubation arrangement.

Thus, the testing device of the invention may have a fan-like a configuration, whereby the different substrates of the plurality of substrates may be attached to each other by a corresponding attachment portion, which may for example correspond to an overlap of mutually attached end portions of the substrates, possibly corresponding to 5% to 20% of the extension of the substrates in the direction of overlap. The fan-like structure of the testing device may open up in the open configuration (like and hand fan), whereby the second substrate may be exposed to the environment, allowing a biological probe to be received therein. In the closed configuration, the fan-like structure of the testing device may close, such that the different substrates of the testing device overlap each other not only in the respective attachment portions, but completely, forming a compact stack.

However, in other embodiments, at least one of the substrates of the plurality of substrates, for example the second substrate, may be separable from the remaining substrates of the plurality of substrates.

In some preferred embodiments, the testing device may further comprise at least one substance pad configured for receiving a respective one of the one or more bioactive substances. The at least one substance pad may in particular be arranged, optionally attached, on one of the substrates of the plurality of substrate other than the second substrate adjacent to the second substrate, such that the at least one substance pad faces the second substrate. For example, the at least one substance pad may be arranged on the first substrate and/or on the third substrate facing the second substrate. The at least one substance pad may additionally or alternatively face any other substrate of the plurality of substrates that may be arranged between the substrate on which the substance pad is arranged and the second substrate. The at least one substance pad may preferably comprise at least one antimicrobial susceptibility disc, for example of the type Fosfmycin 200µm, which may in particular be suitable for being impregnated with the corresponding bioactive substance, for example with an antibiotic drug. Since the at least one substance pad faces the second substrate, a bioactive substance comprised in each of the at least one substance pad may interact with any other substances present in the second substrate and/or in other substrates adjacent thereto by bringing the at least one substance pad in contact with the second substrate and/or with said other substrates. According to this configuration, a testing device according to the invention may be prepared for testing the susceptibility of the biological probe to one given bioactive substance by simply incorporating a substance pad impregnated with said given bioactive substance.

The at least one substance pad, which may in particular comprise one single substance pad, may preferably be arranged facing and/or overlapping an end portion of the second substrate, thereby allowing to extract information about the susceptibility of the biological probe to the corresponding bioactive substance based on how close to the end portion of a substrate impregnated with the bacterial marking substance, e.g. the second substrate, bacterial growth can be observed.

In some preferred embodiments, the testing device may further comprise a fourth substrate arranged or arrangeable between the second substrate and the first substrate or the third substrate, wherein the fourth substrate may be liquid-impregnable, in particular suitable for being impregnated with a bacterial marking substance configured for undergoing a property change in the presence of bacteria. According to this configuration, a testing device according to the invention may be prepared for testing the susceptibility of the biological probe to one or more bioactive substances using a given bacterial marking substance by simply incorporating a fourth substrate impregnated with said given bacterial marking substance.

The fourth substrate may additionally or alternatively comprise or be of an hydrophilic material and/or an adsorbent material, such as paper or cotton, and may be suitable for absorbing and removing excess liquid from the adjacent second substrate.

In some embodiments, the fourth substrate may be further configured for being impregnated with one of the one or more bioactive substances, for example an antibiotic drug, which may in particular be provided in dried form, for example as a powder embedded within the fourth substrate which can dilute into any liquid substance(s) that may be present in the fourth substrate and/or in the second substrate when contacting said liquid substance(s).

In some preferred embodiments, the second substrate may comprise a first partial substrate and a second partial substrate separable from the first partial substrate. Thus, any of the functionalities described herein for the "second substrate" may be implemented by the combination of the first partial substrate and the second partial substrate, which can be separated from each other, i.e. may be structurally independent from each other.

According to one example, the first partial substrate may be liquid-permeable and may be configured for being impregnated with the biological culture medium for supporting bacterial growth. Optionally, the first partial substrate may further be impregnated with one of the one or more bioactive substances and/or with a bacterial marking substance. Preferably, the second partial substrate may be liquid-impregnable, in particular impregnable with the biological probe and/or with one of the one or more bioactive substances, and may correspond to the at least one portion of the second substrate exposable to an environment of the testing device for receiving the biological probe.

Exposing the at least one portion of the second substrate to an environment of the testing device for receiving the biological probe may comprise exposing the second partial substrate to such environment, possibly by separating the second partial substrate from the rest of substrate of the plurality of substrates of the testing device. For example, the second partial substrate may be a separable paper strip that can be brought into contact with a liquid comprising the biological probe, independently of the rest of the testing device, in order to impregnate the second partial substrate with the biological probe, and the second partial substrate can then be rejoined with the first partial substrate, such that the substance(s) present in the first partial substrate and the substance present in the second partial substrate, in particular the biological probe, can mix and react with each other.

According to a further example, the first partial substrate may be liquid-permeable and may be configured for being impregnated with the bacterial marking substance and with one of the one or more bioactive substances and the second partial substrate may be liquid-permeable and may be configured for being impregnated with the biological culture medium and with the biological probe. The second partial substrate may correspond to the at least one portion of the second substrate exposable to an environment of the testing device for receiving the biological probe.

According to some embodiments, the plurality of substrates of the testing device may further comprise a fifth substrate arranged or arrangeable above the remaining substrates of the plurality of substrates, in particular above of the third substrate. The fifth substrate may hence be arranged above all remaining substrates of the plurality of substrates of the testing device in order to be an uppermost substrate of the testing device. The fifth substrate may be radiation shielding, light shielding, thermally insulating and/or air-tight. The fifth substrate may comprise or be made of a material providing any of the aforementioned properties or a combination thereof. For example, the fifth substrate may be made of aluminium and may show all of the aforementioned properties. The fifth substrate may hence constitute a protective substrate or layer configured for protecting the rest of the substrates of the plurality of substrates from degradation and/or contamination and/or any environmental impact. Once a substance, in particular one of the one or more bioactive substances, the biological probe, the biological culture medium and/or the bacterial marking substance, is present in one or more of the remaining substrates of the plurality of substrates of the testing device (other than the fifth substrate), the protection conferred by the fifth substrate can preserve and protect the integrity and durability of such substances.

In some preferred embodiments, the testing device may further comprise a disinfectant reservoir configured for receiving a substance comprising a disinfectant, for example ethanol, an aldehyde, an oxidizing agent and/or a biocide. The disinfectant reservoir may be releasably sealable or sealed by a corresponding sealing element. The sealing element may allow releasing the disinfectant from the disinfectant reservoir when required for disinfecting the testing device or at least a part thereof after use, in particular after obtaining the susceptibility result. The sealing element may for example be a breakable membrane that can be broken by applying a pressure thereon or by bending or twisting the testing device. The disinfectant reservoir may preferably be arranged facing the second substrate and/or any other substrate of the plurality of substrates configured for receiving a biological probe. The disinfectant reservoir provides a simple and safe way of sterilising the testing device after use prior to disposal for bacteriological safety.

In some embodiments of the invention, the testing device may have a "multi-test configuration". This means that the second substrate may comprise one or more probe receiving portions spatially separated from each other and the third substrate may comprise one or more substance reservoirs, wherein each of the one or more substance reservoirs may be configured for receiving one of the one or more bioactive substances and may be releasably sealed by a sealing element. The one or more substance reservoirs may be arranged or arrangeable overlapping at least one of the probe receiving portions, such that, when one of the substance reservoirs is unsealed, the corresponding bioactive substance is released into a corresponding probe receiving portion overlapped by said substance reservoir. The one or more probe receiving portions may be exposed or exposable to the environment of the testing device for receiving the biological probe. The second substrate may preferably be made of a biocompatible material, for example of agar, and may comprise, in particular on a surface of the second substrate opposite to the first substrate, the biological culture medium, and possibly also the bacterial marking substance. The second substrate may be fixedly arranged on the first substrate. The first substrate may laterally protrude from the second substrate, such that a vertical projection of the second substrate may be within a vertical projection of the first substrate. The second substrate, and in particular the probe receiving portions thereof, maybe impregnated with the bacterial marking substance.

Each of the one or more substance reservoirs of the third substrate, which may in particular form a respective cavity, may be configured for receiving one of the one or more bioactive substances to be tested with the testing device and for being releasably sealed by a sealing element. The one or more bioactive substances may be received in the one or more substance reservoirs in liquid, powder or solid form. Further, the one or more bioactive substances may be received in the one or more substance reservoirs by being contained in support elements that can be received in the substance reservoirs. For example, at least some of the one or more substance reservoirs may be configured for receiving a carrying material, preferably an antimicrobial susceptibility disc, containing a corresponding bioactive substance, for example a drug, in particular an antibiotic drug. It is hence possible for each of the one or more substance reservoirs to contain in its interior one of the one or more bioactive substances and to be releasably sealed by a sealing element, such that the respective bioactive substance may be releasably sealed within the corresponding substance reservoir. The one or more bioactive substances can in particular be antibiotic drugs and may preferably be different from each other in one or more of type, composition, dosage, concentration and quantity.

"Releasably sealed" may refer herein to the ability of being sealed such as to form a fluid tight interior while having the ability of being unsealed, for example by a mechanic, electronic, or chemical action upon the corresponding sealing element, for instance by opening, peeling off and/or breaking the sealing element, such as to allow a fluid communication between the interior and the exterior of the corresponding substance reservoir, in particular such as to release a bioactive substance contained in the substance reservoir and allow the bioactive substance to exit the respective substance reservoir in a controlled instant of time.

Each of the one or more substance reservoirs is arranged or arrangeable overlapping at least one of the probe receiving portions such that, when a given one of the substance reservoirs is unsealed, the corresponding bioactive substance, i.e. the bioactive substance that was sealed within said given one of the substance reservoirs, is released into a corresponding probe receiving portion overlapped by said substance reservoir. If, for example, the one or more substance reservoirs are arranged or arrangeable vertically overlapping the at least one of the probe receiving portions, gravity may cause the bioactive substances received in the substance reservoirs to be provided to the corresponding probe receiving portions upon unsealing the substance reservoirs.

Preferably, each of the one or more substance reservoirs may be arranged or arrangeable overlapping a respective one of the probe receiving portions, such that the substance reservoirs and the probe receiving portions are arranged or arrangeable in a one-to-one correspondence, although other configurations are possible, in particular one-to-many and many-to-one configurations. Each of the one or more substance reservoirs may hence be spatially associated with a corresponding one of the probe receiving portions in such a manner that the bioactive substance contained in a given substance reservoir is dispensed into the corresponding probe receiving portion associated with said given substance reservoir when it is released by unsealing said given substance reservoir.

The one or more probe receiving portions may be exposed or exposable to an environment of the testing device for receiving the biological probe, which may in particular be a urine probe or any other fluid probe of a patient. "Exposed" may refer herein to the ability of the probe receiving portions of receiving the biological probe from an environment of the testing device without any other component, in particular the second substrate, being interposed between the probe receiving portions and said environment. The probe receiving portions may be exposed to the environment in a configuration of the testing device corresponding to the previously described "open configuration" and/or the "probe collection arrangement" of the testing device of the invention.

By virtue of the one or more substance reservoirs and the one or more probe receiving portions, the testing device of the invention according to said "multi-test configuration" may allow testing the susceptibility of a biological probe, such as a urine probe or any other fluid probe of a patient, to each of the one or more bioactive substances that are contained in the second substrate and are dispensed to corresponding probe receiving portions using one single testing device, possibly simultaneously. When a biological probe is dispensed to the probe receiving portions of the second substrate, the biological probe impregnates and may diffuse through the respective probe receiving portions of the second substrate, due to the second substrate being liquid-permeable and liquid-impregnable. Bacteria present in the biological probe may then grow fostered by the biological culture medium present in (or added to) the probe receiving portions of the second substrate, thereby triggering a property change of the bacterial marking substance present in (or added to) the probe receiving portions of the second substrate.

If one or more bioactive substances contained in the substance reservoirs are dispensed to corresponding probe receiving portions by unsealing the respective substance reservoirs, in particular shortly after the biological probe is received in the probe receiving portions of the second substrate, said one or more bioactive substances impregnate said corresponding probe receiving portions too and can impair or suppress bacterial growth therein and in a near environment thereof, provided that the bacteria present in the biological probe are not immune, i.e. are susceptible, to the one or more bioactive substances or a part thereof.

If the biological probe contains bacteria that are susceptible to a given one of the bioactive substances impregnating a part of the probe receiving portions of the second substrate, in particular one of the probe receiving portions and a near environment thereof, bacterial growth is impaired at and around such probe receiving portions. As a consequence, there is either no property change of the bacterial marking substance or a less perceivable property change as compared to other probe receiving portions and/or to a probe receiving portion or a part of the probe receiving portions of the second substrate that is not impregnated with any drug and may operate as a negative control. As a result, the bioactive substance or the bioactive substances impregnating parts of the probe receiving portions of the second substrate in which no property change of the bacterial marking substance or only a reduced or partial property change of the bacterial marking substance is observed may be identified as potentially effective bioactive substances for treating a bacterial infection present in the tested biological probe.

If the biological probe contains bacteria that are resistant, i.e. immune, to a given one of the bioactive substances impregnating a part of the probe receiving portions of the second substrate, in particular one of the probe receiving portions and a near environment thereof, bacteria can grow at and around such probe receiving portions, inasmuch as bacterial growth is not impaired by such bioactive substance. As a consequence, there will be a property change of the bacterial marking substance, in particular a property change more pronounced than in the previously mentioned cases in which bacteria are susceptible to a bioactive substance. As a result, the bioactive substance(s) impregnating parts of the probe receiving portions of the second substrate in which the bacterial marking substance undergoes a property change, particularly an increased property change as compared to other parts of the probe receiving portions of the second substrate may be identified as ineffective bioactive substances for treating a bacterial infection present in the tested biological probe and hence discarded as an effective treatment option.

In some preferred embodiments, the third substrate may be movable with respect to the first substrate and/or the second substrate, wherein, in a first position of the third substrate, each of the one or more substance reservoirs is arranged overlapping at least one of the probe receiving portions and wherein, in a second position of the third substrate, the one or more probe receiving portions are exposed to an environment of the testing device, in particular by the third substrate. In the first position of the third substrate, it is possible to release the bioactive substances received in the substance reservoirs such as to dispense the bioactive substances into the corresponding probe receiving portions, for example by direct contact or by the effect of gravity. In the second position, the probe receiving portions of the second substrate are exposed by the third substrate such that it is possible to receive a biological probe into the probe receiving portions of the second substrate. The third substrate may for example be removably attached or attachable to the first substrate and/or to the second substrate. The third substrate may in particular be flexibly or pivotably attached to the first substrate and/or to the second substrate, such that the third substrate may be movable between the first position and the second position.

According to some embodiments, at least some of the one or more probe receiving portions may be separated from each other at least partly by a fluid tight isolating element. The isolating element may preferably be hydrophobic, for example may comprise a wax or any other hydrophobic material. The isolating element may be used to ensure fluid separation between the different probe receiving portions such as to guarantee the reliability of the drug susceptibility results obtained with the testing device.

In some preferred embodiments, at least a part of the probe receiving portions may be arranged regularly and/or symmetrically with respect to each other, in particular on the first substrate. For example, said at least a part of the probe receiving portions may be evenly arranged on a (virtual) circumference on the first substrate, or may be arranged forming a regular polygonal structure on the substrate such that each of the probe receiving portions occupies a vertex of the polygonal structure. A regular and/or symmetrical arrangement of the probe receiving portions may provide correspondingly regular testing conditions for the different bioactive substances and hence improve the comparability of the results obtained for each of the different bioactive substances tested with the testing device. However, other probe receiving portions may be arranged on the substrate not belonging to such regular and/or symmetrical formations.

Preferably, said at least a part of the probe receiving portions may be arranged around a common central section of the probe receiving portions of the second substrate and may be connected therewith by respective connection arms of the probe receiving portions of the second substrate. The connection arms may hence be elongated portions of the probe receiving portions of the second substrate respectively connecting one of the probe receiving portions with another probe receiving portion and/or with said common central section of the probe receiving portions of the second substrate. For example, if said at least a part of the probe receiving portions are arranged on the vertices of a regular polygon, the respective connection arms may extend along respective circumradii of the polygon connecting a corresponding probe receiving portion with the common central section, such that all connection arms may meet at the central point of said polygon.

If a probe receiving portion contains a bioactive substance to which the biological probe is not susceptible, i.e. is immune, bacterial proliferation will not be suppressed in that probe receiving portion, for which bacteria may grow from that probe receiving portion and extend into the connection arm joining that probe receiving portion with the common central section. In contrast, if a probe receiving portion contains a bioactive substance to which the biological probe is susceptible, i.e. is not immune, bacterial proliferation will be suppressed in that probe receiving portion, for which bacteria shall not grow extending into the connection arm joining that probe receiving portion with the common central section or will extend to a smaller extent. The susceptibility of the biological probe to the one or more bioactive substances received in the different probe receiving portions may be easily compared by comparing bacterial growth into the different connection arms. For example, if the bacterial marking substance is a chromogenic substance configured for undergoing a change in colouration in the presence of bacteria, the susceptibility of the biological probe to the one or more bioactive substances may be easily compared visually my comparing the extension of the portions of the connection arms that display a change in colouration.

In some embodiments, one of the probe receiving portions may be arranged at said common central section of the probe receiving portions of the second substrate as a central probe receiving portion. The central probe receiving portion may be arranged among said at least a part of the probe receiving portions and may be connected therewith by the respective connection arms of the probe receiving portions of the second substrate. Thus, said at least one part of the probe receiving portions may be regularly and/or symmetrically arranged around the central probe receiving portion. For example, if said at least one part of the probe receiving portions is arranged on the vertices of a regular polygon, the central probe receiving portion may be arranged at the centre of the polygon and the respective connection arms may extend along respective circumradii of the polygon connecting a corresponding probe receiving portion with the central probe receiving portion.

In some embodiments, the probe receiving portions of the second substrate may further comprise at least one dummy probe receiving portion fluidly connected to one or more of the one or more probe receiving portions, preferably to each one of them. The at least one dummy probe receiving portion maybe arranged or arrangeable not being overlapped by any substance reservoir or may be arranged or arrangeable being overlapped by a substance reservoir which does not contain any bioactive substance. The aforesaid central probe receiving portion may correspond to one such dummy probe receiving portion.

Thus, irrespectively of the number and arrangement of the probe receiving portions of the second substrate, the probe receiving portions of the second substrate may comprise one or more probe receiving portions, for instance dummy probe receiving portions, which are not arranged or arrangeable being overlapped by any substance reservoir or are arranged or arrangeable being overlapped by a substance reservoir which does not contain any bioactive substance. Such one or more probe receiving portions, which may in particular comprise the aforementioned central probe receiving portion, may be used as a positive or negative control probe receiving portion to confirm or discard the presence of bacteria in a biological probe dispensed to the probe receiving portions of the second substrate. If a biological probe dispensed to the probe receiving portions of the second substrate contains bacteria, this can be detected by such probe receiving portions being used as a positive or negative control by a corresponding property change of the bacterial marking substance received therein.

In some embodiments, the third substrate may further comprise at least one dummy reservoir arranged or arrangeable overlapping the at least one dummy probe receiving portion and configured for receiving a substance therein and being releasably sealed by a corresponding sealing element. The at least one dummy probe receiving portions, which may in particular comprise or correspond to the aforementioned central probe receiving portion, may be used as a positive or negative control probe receiving portion to confirm or discard the presence of bacteria in a biological probe dispensed to the probe receiving portions of the second substrate, in particular without any bioactive substance interaction. If a biological probe dispensed to the probe receiving portions of the second substrate contains bacteria, this will be detected by such probe receiving portions being used as a positive or negative control by a corresponding property change of the bacterial marking substance.

Further, the at least one dummy reservoir of the third substrate may be used to receive therein a substance other than a bioactive substance for interaction with the biological probe, in particular after the susceptibility results have been obtained for the one or more bioactive substances. For example, said substance may in particular be or comprise a disinfectant. This allows easily dispensing the disinfectant, by non-trained users, into the probe receiving portions of the second substrate for biological safety after use of the testing device of the invention for sterilising the probe receiving portions of the second substrate or a part thereof impregnated with the biological probe and the one or more bioactive substances. The disinfectant may for example be or comprise ethanol. The disinfectant may be released from the at least one dummy reservoir into a corresponding at least one dummy probe receiving portion, for example into a central probe receiving portion acting as a probe receiving portion, and spreading from there through all parts of the probe receiving portions of the second substrate that are fluidly connected with said at least one dummy probe receiving portion.

Irrespectively of the number and arrangement of the probe receiving portions of the second substrate, all or some portions of the probe receiving portions of the second substrate, in particular the probe receiving portions and possibly the respective connection arms and/or the dummy probe receiving portions, may be in fluid communication with each other, possibly indirectly (e.g. via connection arms and a central probe receiving portion) in such a manner that it is possible for the biological probe to propagate through the probe receiving portions of the second substrate and impregnate it when the biological probe is administered to the probe receiving portions of the second substrate. However, the invention also foresees embodiments in which at least some portions of the probe receiving portions of the second substrate may be isolated, in particular fluidly isolated, from other portions of the probe receiving portions of the second substrate.

Additionally or alternatively, the third substrate may further comprise at least one disinfectant reservoir configured for receiving a disinfectant, preferably ethanol, wherein the at least one disinfectant reservoir is releasably sealed by a corresponding sealing element, wherein the disinfectant reservoir is arranged or arrangeable overlapping a volume in fluid communication with each of the probe receiving portions or a part thereof. Thus, when the disinfectant reservoir is unsealed, the disinfectant is released into a volume in fluid communication with each or a part of the probe receiving portions. It is hence possible for the disinfectant to be dispensed into the probe receiving portions by other means than through diffusion from a dummy or central probe receiving portion, in particular by being directly dispensed through a volume in fluid communication with each or a part of the probe receiving portions. For example, the at least one disinfectant reservoir may be arranged above the probe receiving portions of the second substrate at an overhead distance from the probe receiving portions of the second substrate, such that a gap is formed between the at least one disinfectant reservoir and the probe receiving portions of the second substrate and the amount of disinfectant in the at least one disinfectant reservoir may be enough to fill said gap upon being released such as to totally or partly sterilize the probe receiving portions of the second substrate.

The at least one disinfectant reservoir may be configured for unsealing by folding and/or pressing the testing device together, such that it is in particular possible to sterilize the testing device after use and before disposal by simply folding and/or pressing the testing device together.

In preferred embodiments of the invention, at least some of the probe receiving portions, in some cases all of the probe receiving portions may be equal in size and/or shape. This may improve the direct comparability of the susceptibility results obtained with the test device for the different bioactive substances. The probe receiving portions may in particular be circular, oval or polygonal.

In some embodiments, at least one of the one or more substance reservoirs of the third substrate may comprise a first cavity and a second cavity. The first cavity may be releasably separated from the second cavity by an intermediate sealing element, for example by an intermediate membrane or sealing sheet. The intermediate sealing element may provide a removable or breakable separation between the first cavity and the second cavity, such that the separation between the first cavity and the second cavity may be broken or removed, in particular by means of a mechanical, chemical or electronic action upon the respective substance reservoir, for example by applying a mechanical force. In such embodiments, it may be possible to receive a powder or solid bioactive substance into one of the first and second cavity and a liquid solvent in the other one of the first and second cavity, such that, the powder or solid bioactive substance and the liquid solvent may come in contact with one another upon removing the separation between the first cavity and the second cavity. The use of powder or solid bioactive substances in the testing device of the invention may allow extending the lifespan of the testing ability of the testing device, due to the longer durability of powder or solid bioactive substances as compared to liquid/dissolved bioactive substances.

In some embodiments, at least one of the one or more substance reservoirs (possibly including one or more dummy reservoirs) may be releasably sealed by a corresponding individual releasable sealing element, preferably by a breakable sealing element and more preferably via a pressure-sensitive and/or a radiation-shielding breakable sealing element. The individual releasable sealing elements may allow selectively unsealing individually substance reservoirs, such that it may be possible to unseal one of the substance reservoirs, some or all of the substance reservoirs. If the sealing elements are breakable sealing elements, the substance reservoirs may be unsealed by breaking the sealing elements, for example by applying a mechanical force. If the sealing elements are pressure-sensitive, the substance reservoirs may be unsealed by simply applying a pressure, for example by pressing with a hand or a finger of a patient or user of the testing device. If the sealing elements are radiation-shielding, this may allow better preserving a bioactive substance received in the substance reservoirs, thereby extending the durability of the testing device.

In some embodiments, at least some of the substance reservoirs, possibly all of the substance reservoirs, may be releasably sealed by a common releasable sealing element. The releasable sealing element may be a removable sealing element, for example a peelable membrane, and may be preferably made of a radiation-shielding material. If the sealing element is a common releasable sealing element, said at least some of the substance reservoirs may be unsealed in one go by simply releasing the releasable sealing element, for example by removing the releasable sealing element.

According to some embodiments, the testing device of the invention according to said "multi-test configuration" may further comprise a separation sheet arranged or arrangeable between the third substrate and the first substrate and/or the probe receiving portions of the second substrate. The separation sheet may comprise one or more openings, wherein each of the openings may overlap one of the one or more probe receiving portions. Thus, the one or more substance reservoirs of the third substrate may be arranged or arrangeable overlapping corresponding probe receiving portions through the respective openings of the separation sheet, such that, when a substance reservoir is unsealed, the bioactive substance received therein is dispensed to the underlying probe receiving portion or probe receiving portions through a corresponding opening of the separation sheet. The separation sheet, which may preferably be of a plastic material, may protect the probe receiving portions of the second substrate during the manipulation of the testing device, for example protection against mechanical damage, heat and/or humidity. The separation sheet may however be permeable to oxygen and/or humidity.

In some embodiments, the testing device according to said "multi-test configuration" may further comprise a fifth substrate configured as a protective foil for covering and/or wrapping the test device at least partially, possibly completely, in particular the second substrate. The protective foil is preferably radiation-shielding, light-shielding, thermally insulating, elastic, fluid-tight and/or air-tight. The protection provided by the protective foil arranged on the second substrate may preserve the bioactive substances contained in the third substrate and/or second substrate from degradation and hence extend the durability of the testing device. Further, if used for wrapping the testing device, in particular after use, the protective foil may fluid-tightly isolate the interior of the testing device from the environment, thereby protecting the environment from bacteriological contamination.

A further aspect of the invention refers to a testing kit for testing the susceptibility of a biological probe to one or more bioactive substances. The testing kit comprises a testing device according to any of the previously described embodiments and at least one of: one or more bioactive substances, a biological culture medium, and a bacterial marking substance configured for undergoing a property change in the presence of bacteria.

In preferred embodiments, the second substrate of the testing device and/or any of the other substrates of the plurality of substrates of the testing device arranged adjacent to the second substrate may be impregnated with an activator of the one or more bioactive substances for increasing an effectivity thereof, for example with Glucose-6-phosphate.

The testing kit of the invention hence comprises not only the testing device, but also one or more bioactive substances with respect to which the susceptibility of a biological probe dispensed to the testing device is to be tested, a biological culture medium, and/or a bacterial marking substance configured for undergoing a property change in the presence of bacteria. The testing kit of the invention can thus form a portable easy-to-use POCT, which may be in particular configured as a patch-like/strip-like testing kit.

In some embodiments, the testing kit of the invention may further comprise an inhibiting substance configured for inhibiting one or more predefined probe components present in a biological probe. The inhibiting substance, which may correspond to the "inhibiting substance" described above with respect to the testing device of the invention, may be comprised in one or more of the substrates of the plurality of substrates of the testing device of the testing kit of the invention, in particular in a substrate of the plurality of substrates arrangeable next to the second substrate. Thereby, unwanted components of a biological probe to be tested that may possibly affect or falsify the results of the susceptibility test can be inhibited before performing a susceptibility test using the testing device of the present invention, thus reducing the risk of false results. Additionally or alternatively, the testing kite may comprise a filtering device configured for filtering out from the biological probe the one or more predefined probe components.

The one or more bioactive substances maybe or comprise antibiotics, for example amoxicillin, ciprofloxacin, levofloxacin, nitrofurantoin, pivmelam, monuril, cefuroxime, nitroxolin and/or cotrimoxazol. The one or more bioactive substances may all correspond to drugs potentially suitable to treat a particular type of bacterial infection, for example urinary tract infection (UTI). However, the invention is not limited to the use of drugs or antibiotics and the one or more bioactive substances may also be or comprise other types of bioactive substances such as biocides or fungicides.

The one or more bioactive substances of the testing kit may preferably be different form each other, in particular in one or more of type, composition, dosage, concentration and quantity.

According to some embodiments, a portion of the second substrate of the testing device of the testing kit, preferably an end portion thereof, may comprise and/or be impregnated with one of the one or more bioactive substances. Additionally or alternatively, a portion of a further substrate of the testing device adjacent to the second substrate, preferably an end portion thereof, may comprise and/or be impregnated with said one of the one or more bioactive substances. For example, the second substrate may have a strip-like elongated shape and one bioactive substance maybe arranged at one longitudinal end of the strip-like elongated shape. According to such configurations, it may be possible to infer a degree of susceptibility or resistance of bacteria present in the biological probe to the bioactive substance being tested based on how close to said end portion of the second substrate in which the one or more bioactive substances are arranged (e.g. how close to said longitudinal end of the strip-like elongated shape of the second substrate) bacterial growth is signaled by the bacterial marking substance.

Further, the bacterial marking substance may be configured for undergoing a different property change in the presence of different types of bacteria. For example the bacterial marking substance may be a chromogenic substance configured for taking a different coloration for different types of bacteria. For instance, a chromogenic substance may turn red in the presence of E. coli bacteria while it may turn blue in the presence of Staph. Saprophyticus bacteria, such that the colour taken up by the chromogenic substance may allow inferring information about the type of bacteria present in the biological probe, whereas the colour intensity may provide information about the concentration of the corresponding type of bacteria.

The one or more bioactive substances, in particular one bioactive substance, may thus be arranged or arrangeable at said end portion of the second substrate, preferably in dry form, for example as a powder embedded in the second substrate or in a substrate adjacent thereto. When the fluid biological probe contacts the bioactive substance, the bioactive substance may dilute in the biological probe and may locally impregnate the second substrate, locally impairing bacterial growth around an original location of the bioactive substance.

In preferred embodiments, the second substrate of the testing device of the testing kit of the invention may comprise and/or be impregnated with the bacterial marking substance. Additionally or alternatively, a further substrate of the testing device adjacent to the second substrate may comprise and/or be impregnated with the bacterial marking substance.

According to preferred embodiments of the invention, the testing device may comprise at least one substance pad comprising or impregnated with a respective one of the one or more bioactive substances. The at least one substance pad may in particular be arranged on one of the substrates of the plurality of substrates other than the second substrate and adjacent to the second substrate, in particular on the first substrate and/or on the third substrate, such that the at least one substance pad faces the second substrate. The at least one substance pad may preferably comprise at least one antimicrobial susceptibility disc comprising or being impregnated with the respective one of the one or more bioactive substances. The use of an antimicrobial susceptibility disc may provide a longer life span for the corresponding bioactive substances. Since the at least one bioactive substance is comprised in the at least one substance pad facing the second substrate, the at least one substance pad may contact the second substrate, for example when the testing device is arranged in the closed configuration and/or in the incubation arrangement described above, whereby the at least one bioactive substance can react with the other substance(s) present in the second substrate or flowing to the second substrate from other adjacent substrates, in particular with the biological probe.

In some embodiments, the testing device may further comprise a fourth substrate arranged or arrangeable between the second substrate and the first substrate or the third substrate. The fourth substrate may comprise and/or be impregnated with the bacterial marking substance. Additionally or alternatively, a portion of the fourth substrate, preferably an end portion, may comprise and/or be impregnated with the one of the one or more bioactive substances. The fourth substrate may in particular be arranged or arrangeable adjacent to the second substrate. Thus, the fourth substrate can contact the second substrate, for example when the testing device is arranged in the closed configuration and/or in the incubation arrangement described above, whereby the at least one bioactive substance can react with the other substance(s) present in the second substrate or flowing to the second substrate from other adjacent substrates, in particular with the biological probe and possibly with the bacterial marking substance.

The bacterial marking substance may in particular be or comprise a chromogenic substance configured for undergoing a change in colouration in the presence of bacteria, such that when bacteria grow in contact with the bacterial marking substance, such bacterial growth is signalled by a change of colouration of the chromogenic substance, where a colour intensity may be indicative or the concentration of bacteria. The chromogenic substance may for example be or comprise a pH sensitive dye or a nitrite sensitive dye.

According to preferred embodiments of the invention, the testing device of the testing kit may comprise a disinfectant reservoir containing a substance comprising or being a disinfectant. The disinfectant may preferably be or comprise ethanol. The disinfectant reservoir may be releasably sealed by a corresponding sealing element. The disinfectant reservoir may preferably be arranged facing the second substrate of the testing device and/or any other substrate of the plurality of substrates of the testing device configured for receiving a biological probe. The disinfectant reservoir provides a simple and safe way of sterilizing the testing device after use prior to disposal for bacteriological safety. Once the testing kit has been used for obtaining one or more susceptibility results, the disinfectant can be released from the disinfectant reservoir and used to sterilize the testing device prior to disposal.

In some embodiments of the testing kit of the invention, testing device of the testing kit may be a testing device according to any of the embodiments of said "multi-test configuration" described above, wherein at least some of the one or more substance reservoirs of the testing device may contain one of the one or more bioactive substances, wherein the one or more bioactive substances may preferably be different from each other.

At least some of the probe receiving portions and/or at least some of the connection arms of the second substrate of the testing device may have an homogeneous concentration of the biological culture medium and/or of the bacterial marking substance. This may further improve the comparability of the susceptibility results obtained with the test device for the different bioactive substances.

In some embodiments, the at least one of the one or more substance reservoirs of the testing device may comprise a first cavity and a second cavity, wherein the first cavity may be releasably separated from the second cavity by an intermediate sealing element. The corresponding bioactive substance may be a powder or solid substance contained in the first cavity and a liquid solvent may be contained in the second cavity. The use of powder or solid drugs in the testing kit of the invention may allow extending the lifespan of the testing kit, due to the longer durability of powder or solid substances as compared to liquid/dissolved substances, which applies in particular to drugs. The liquid solvent may for example be or comprise water, in particular distilled water, acetone, chloroform and/or dimethyl sulfoxide (DMSO).

In some embodiments, at least one of the one or more substance reservoirs may comprise, in particular instead of directly comprising the corresponding bioactive substance as a fluid, powder or solid substance by itself, a substance pad, preferably an antimicrobial susceptibility disc, containing the corresponding bioactive substance. The use of an antimicrobial susceptibility disc may provide a longer life span and/or more stable environmental conditions for the corresponding bioactive substances to be tested.

In some embodiments of the testing kit of the invention comprising a testing device according to the previously described "multi-test configuration", at least one of the substance reservoirs of the testing device may contain a substance comprising or being a disinfectant, for example ethanol.

In some embodiments of the invention, the testing kit may further comprise a probe recipient configured for receiving a liquid containing a biological probe. The at least one portion of the second substrate of the testing device may be insertable into the probe recipient, in particular when the biological probe is received in the probe recipient, for being impregnated with the biological probe. The probe recipient may for example be a plastic or paper can usable by a patient for depositing therein a liquid comprising the biological probe, for example a urine probe, and for depositing the biological probe on the second substrate, for example by dipping the exposable portion of the second substrate into the probe recipient filled with the liquid comprising the biological probe.

A further aspect of the invention refers to a method of testing the susceptibility of a biological probe to one or more bioactive substances using a testing device or a testing kit according to any of the previously described embodiments.

The method of the invention comprises, in particular: dispensing the biological probe into at least a portion of the second substrate of the testing device, and reacting the biological probe with the one or more bioactive substances in the presence of a bacterial marking substance, preferably in the second substrate of the testing device, wherein the bacterial marking substance is configured for undergoing a property change in the presence of bacteria.

The method may further comprise exposing the at least one portion of the second substrate of the testing device before dispensing the biological probe and then dispensing the biological probe into said at least one portion of the second substrate being exposed. The at least one portion of the second substrate may be exposed by arranging the testing device in a configuration corresponding to the previously described "open configuration" and/or the "probe collection arrangement".

For example, the at least one portion of the second substrate may be exposed to an environment of the testing device by separating the second substrate from the remaining substrates of the plurality of substrates of the testing device. In embodiments in which the second substrate is movable with respect to the remaining substrates and and/or probe receiving portions of the second substrate between a first (closed) position of the second substrate, in which the one or more substance reservoirs are arranged overlapping at least one of the probe receiving portions, and a second (open) position of the second substrate, in which the one or probe receiving portions are exposed to an environment of the testing device, exposing at least a part of the probe receiving portions of the second substrate may comprise setting the probe receiving portions of the second substrate in the second (open) position.

Dispensing the biological probe into at least a portion of the second substrate may comprise exposing the at least one portion of the second substrate to a flow of liquid comprising the biological probe or soaking the at least one portion of the second substrate with said liquid, preferably with said liquid being received in a probe recipient. For example, dispensing the biological probe into the at least one portion of the second substrate, which may be previously exposed, may for example comprise collecting in the second substrate a urine probe of a patient directly during urination or by soaking the second substrate or a further substrate into a urine probe contained in a probe recipient of the testing kit of the invention.

The method of the invention may further comprise, after dispensing the biological probe into the at least one portion of the second substrate of the testing device and before reacting the biological probe with the one or more bioactive substances, arranging the testing device in a configuration corresponding to the previously described "closed configuration" and/or the "incubation arrangement", such that the second substrate is protected from an environment of the testing device, in particular by the remaining substrates of the plurality of substrates.

In some preferred embodiments, the method may further comprise attaching the test device to a body of a patient. Thereby, the biological probe can be incubated using the warmth and humidity produced by said body, such that the incubation may take place anywhere, inasmuch as a patient can use his/her own body for incubation, for example by attaching the test device to his/her lap.

In some embodiments, the method of the invention may further comprise inhibiting one or more predefined probe components present in a biological probe, for example enzymes, before and/or while reacting the biological probe with the one or more bioactive substances. The inhibiting may comprise filtering the biological probe with a filtering device configured for filtering out the one or more predefined probe components and/or applying to the biological probe an inhibiting substance configured for inhibiting the one or more predefined probe components present in a the biological probe.

According to some embodiments, the method of the invention may further comprise incubating the biological probe in contact with the one or more bioactive substances. During incubation, which may in typical examples last up to 24 hours but may in some cases be longer, for example between 24 hours and 72 hours, the biological probe, and in particular bacteria contained therein, may grow in the probe receiving portions of the second substrate fostered by the biological culture medium, wherein bacterial growth may be suppressed in regions of the probe receiving portions of the second substrate impregnated by a bioactive substance to which the bacteria contained in the biological probe are susceptible. The incubation may take place in a relatively small and simple incubator without necessarily requiring transportation to specialized laboratory facilities.

According to some embodiments, the method may further comprise wrapping at least a part of the testing device, in particular at least the second substrate and preferably the entire test device, in particular using a protective foil of the testing device and preferably before use. The protective foil may correspond to the previously described "fifth substrate" of the testing device of the invention. The testing device may be wrapped in the protective foil before use, for example during its shelf life. The protective foil may preferably be radiation-shielding, light-shielding, thermally insulating, elastic, fluid-tight and/or air-tight.

Although most of the exemplary explanations of the testing device and a testing kit of the present invention described above are referred to their use for testing the susceptibility of a biological probe comprising urine to one or more antibiotic drugs, the testing device and a testing kit of the invention can also be used for testing the susceptibility of other types of biological probes to different types of bioactive substances. For example, it is possible to use the testing device and the testing kit of the present invention to test the susceptibility of microbes found in households, clinical environments, industrial environments, houses of hyper-allergic persons, border control or shipped goods to different types of biocide substances. Further, it is possible to use the testing device and the testing kit of the present invention to test the susceptibility of biological probes to one or more fungicides. The testing device and the testing kit of the present invention may also be employed for animal farming and veterinary medicine in general.

### BRIEF DESCRIPTION OF THE FIGURES

The description will be described in the following with reference to preferred embodiments thereof as represented in the Figures.
- Fig. 1: shows a schematic side view of a testing device according to embodiments of the invention.
- Fig. 2: shows a schematic side view of the testing device of Fig. 1 in a different arrangement.
- Fig. 3: shows a schematic side view of a testing device according to related embodiments of the invention.
- Fig. 4: shows a schematic side view of a testing device according to related embodiments of the invention.
- Fig. 5: shows a schematic side view of a testing device according to related embodiments of the invention.
- Fig. 6: shows a schematic top view of a testing device of a testing kit according to a related embodiment of the invention showing an antibiogram.
- Fig. 7: shows a schematic top view of a testing device of a testing kit according to a related embodiment of the invention showing a antibiogram.
- Fig. 8: shows a schematic top view of a testing device of a testing kit according to a related embodiment of the invention showing a antibiogram.
- Fig. 9: shows a schematic side view of a testing device according to related embodiments of the invention.
- Fig. 10: shows a schematic side view of the testing device of Fig. 9 in a different arrangement.
- Fig. 11: shows a schematic top view of the testing device of Fig. 9 and 10.
- Fig. 12: shows a schematic top view of the testing device of Fig. 9 and 10.
- Fig. 13: shows a schematic side view of a testing device according to embodiments of the invention.
- Fig. 14: shows a schematic side view of the testing device of Fig. 13 in a different configuration.
- Fig. 15: shows a schematic side view illustration of the testing device of Fig. 13 and 14 in a different configuration.
- Fig. 16: shows a result obtained with a testing kit according to a related embodiment of the invention.
- Fig. 17: shows a flow diagram schematically illustrating a method of testing the susceptibility of a biological probe to one or more bioactive substances using a testing device according to embodiments of the invention.

### DESCRIPTION OF PREFERRED EMBODIMENTS

For the purposes of promoting an understanding of the principles of the invention, reference will now be made to preferred embodiments illustrated in the drawings, and specific language will be used to describe the same. It will nevertheless be understood that no limitation of the scope of the invention is thereby intended, such alterations and further modifications in the illustrated apparatus and such further applications of the principles of the invention as illustrated therein being contemplated as would normally occur now or in the future to one skilled in the art to which the invention relates.

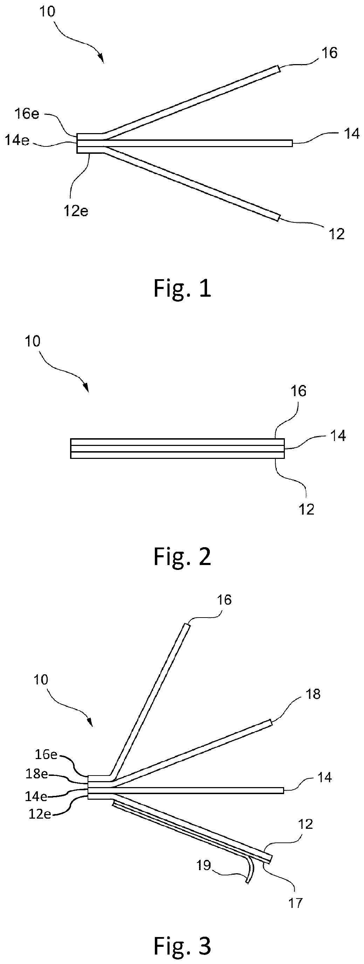

Fig. 1 and 2 show schematic side views of a testing device 10 according to an embodiment of the invention comprising a first substrate 12, a second substrate 14 and a third substrate 16. The second substrate 14 is a liquid-permeable and liquid-impregnable substrate made of paper, which is sandwiched between the first substrate 12 and the third substrate 16, which are both made of a liquid-impermeable plastic material.

As shown in Fig. 1, the substrates 12, 14 and 16 are configured as different substrate-layers, each having a thickness of about 1 mm. The testing device has a fan-like configuration: the substrates 12, 14 and 16 are mutually bound by respective overlapping end portions 12e, 14e, 16e, at which the substrates 12, 14 and 16 remain attached to each other when the testing device 10 is arranged in an open configuration (cf. Fig. 1), whereby the second substrate 14 can be exposed to an environment of the testing device 10, for example for collecting a biological probe to be tested on the second substrate 14. This configuration also corresponds to the "probe collection arrangement" described above. Since the second substrate 14 is liquid-permeable and liquid-impregnable, a liquid contacting the second substrate 14, in particular a liquid containing or being the biological probe, can diffuse through the second substrate 14 impregnating the entire second substrate 14.

The testing device 10 can also be arranged in a closed configuration, shown in Fig. 2, in which the substrates 12, 14 and 16 completely overlap, such that the first substrate 12 and the third substrate 16 tightly enclose the second substrate 14 and protect the second substrate 14 from the surrounding environment. This configuration also corresponds to the "incubation arrangement" described above. Since the first substrate 12 and the third substrate 16 are both liquid-impermeable, they form respective liquid barriers that prevent any liquid impregnating the second substrate 14 from escaping from the second substrate 14, although the first substrate 12 and/or the third substrate 16 may be permeable to gases, for example to oxygen and/or to humidity (water vapour).

The first substrate 12 and the third substrate 16 prevent bacterial migration therethrough, i.e. prevent bacteria from outside the testing device from reaching the interior of the testing device 10, in particular the second substrate 10, and can further prevent bacteria from the second substrate 14 from escaping through the first substrate 12 and/or the third substrate 16.

The second substrate 14 of the testing device 10 illustrated in Figs. 1 and 2 can be used for receiving therein a biological culture medium for supporting bacterial growth, a bacterial marking substance configured for undergoing a property change in the presence of bacteria, a sample of the bioactive substance such as an antibiotic drug or a fungicide, and a biological probe, the susceptibility of which is to be tested. All the aforementioned substances can impregnate the second substrate 14 and react with each other in the second substrate 14.

Fig. 3 shows a schematic side view of a testing device 10 according to a related embodiment, wherein the testing device 10 comprises, apart from the first substrate 12, the second substrate 14 and the third substrate 16, a fourth substrate 18 arranged between the third substrate 16 and the second substrate 14. The testing device 10 of the embodiment illustrated in Fig. 3 also has the previously described fan-like configuration, wherein the open configuration is illustrated in Fig. 3. The substrates 12, 14, 16 and 18 are mutually bound by respective overlapping end portions (cf. substrate ends in Fig. 3).

The fourth substrate 18 of the exemplary embodiment illustrated in Fig. 3 is arranged adjacent to the second substrate 14 and can be made of a liquid-permeable material, for example porous paper having a pore size of 0.1 µm. The fourth substrate 18 can be used for receiving therein the bacterial marking substance and a sample of the bioactive substance, for example in dry or powder form, which then need not be comprised in the second substrate 14. In that case, the second substrate 14 can comprise the biological culture and can receive and be impregnated with the biological probe. When the testing device 10 of the embodiment illustrated in Fig. 3 is arranged in the closed configuration, the second substrate 14 and the fourth substrate 18 contact each other, thereby causing the biological probe in the second substrate 14 to react with the bacterial marking substance and the bioactive substance in the fourth substrate 18. Also in this case, the reaction of the bacterial marking substance provides information about the susceptibility of the bacteria in the biological probe to the bioactive substance.

In the exemplary embodiment illustrated in Fig. 3, the first substrate 12 of the testing device 10 is further coated with a layer of adhesive material 17, which is protected by a peelable protection film 19. By removing the protection film 19, the adhesive material 17 is exposed and can be used for attaching the testing device 10, by the first substrate 12, to a substrate, in particular to the skin of a patient from which the biological probe has been obtained. When attached to the skin of the patient, the warmth and humidity produced by the body of the patient can be used for incubating the biological probe in the testing device 10.

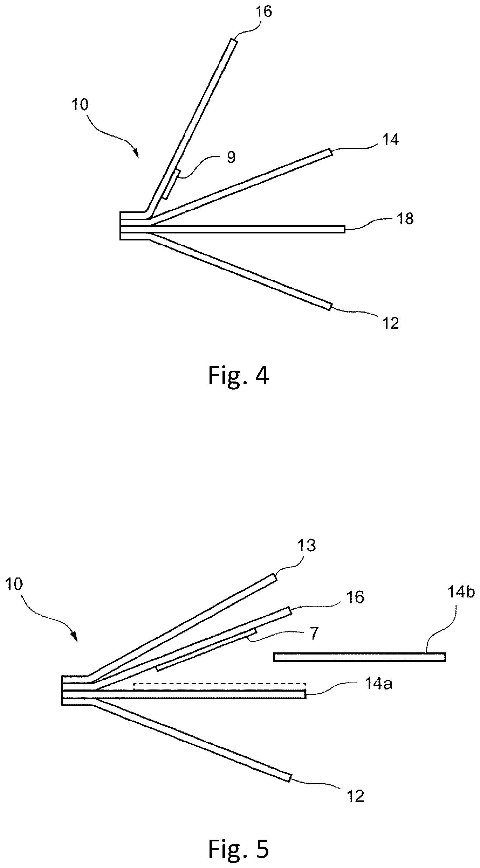

Fig. 4 shows a schematic side view of a testing device 10 according to a related embodiment of the invention. In this case, the order of the second substrate 14 and the fourth substrate 18 is reversed with respect to the arrangement of the embodiment illustrated in Fig. 3. Further, the testing device 10 comprises a substance pad 9 that is arranged on the third substrate 16 facing the second substrate 14. The substance pad 9 is arranged proximal to one of the end portions of the third substrate 16 (close to the left end portion in Fig. 4).

In this configuration, the fourth substrate 18 can be a substrate made of a hydrophilic material, for example cotton, and can be configured for absorbing an excess liquid and/or humidity from the second substrate 14, which can be impregnated with the biological culture medium, the bacterial marking substance and the biological probe. The bioactive substance to be tested can be impregnated in the substance pad 9, which can be advantageous for first manufacturing a large number of testing devices 10 having the same structure and then incorporating selected bioactive substances to be tested to each of the testing devices 10 using a corresponding substance pad 9.

When the testing device 10 of the embodiment illustrated in Fig. 4 is arranged in the closed configuration (not shown), the substance pad 9 contacts the second substrate 14, whereby the bioactive substance comprised in the substance pad 9 can react with the bacteria in the biological probe impregnating the second substrate 14.

Fig. 5 shows a schematic side view of a testing device 10 according to a related embodiment of the invention, wherein the testing device 10 comprises a fifth substrate 13 made of aluminium that is arranged above the third substrate 16, i.e. above all other substrates of the testing device 10, for providing radiation shielding, light shielding, thermal insulation and air-tightness to the testing device 10 in order to protect the substances that are comprised in the underlying substrates.

In the testing device 10 of the exemplary embodiment illustrated in Fig. 5, the second substrate 14 comprises a first partial substrate 14a and a second partial substrate 14b. The first partial substrate 14a is integrated in the fan-like configuration of the testing device 10 by being bounded with the remaining substrates of the testing device 10 (apart from the second partial substrate 14b) by corresponding overlapping end portions of the substrates. The second partial substrate 14b can be separated from the first partial substrate 14a.

The second partial substrate 14b can be a strip of bare absorbent paper (not impregnated a priori with any substance), which confers to the second partial substrate 14b liquid-impregnable properties. Therefore, the second partial substrate 14b can be used for collecting a liquid sample of the biological probe by separating the second partial substrate 14b and exposing it to the biological probe, for example by dipping the second partial substrate 14b into a recipient containing the liquid sample or by directly exposing the second partial substrate 14b to a flow of the liquid sample. When exposed to the liquid sample, the second partial substrate 14b is impregnated with liquid sample comprising the biological probe and can then be arranged on the first partial substrate 14a as indicated by dashed lines in Fig. 5.

The first partial substrate 14a can be impregnated with a biological culture medium and with the bacterial marking substance. A sample of a bioactive substance to be tested can be comprised in the first partial substrate 14a or added thereto, for example from a substance pad 9 (not shown) similar to the substance pad 9 described for the exemplary embodiment illustrated in Fig. 4.

Thus, once the second partial substrate 14b is arranged on the first partial substrate 14a and a testing device 10 is arranged in the closed configuration, all substances present in the first partial substrate 14a and in the second partial substrate 14b can mix and react with each other during incubation.

The testing device 10 of the exemplary embodiment illustrated in Fig. 5 further comprises a disinfectant reservoir 7 arranged on the third substrate 16 facing the first and second partial substrates 14a, 14b of the second substrate 14. The disinfectant reservoir 7 contains ethanol sealed by a breakable membrane (not shown). After the use of the testing device 10, and before its disposal, the breakable membrane of the disinfectant reservoir 7 can be broken by pressing, bending and/or twisting the testing device 10, thereby causing the disinfectant contained in the disinfectant reservoir 7 to disperse onto the first and second partial substrates 14a, 14b, thereby sterilising the testing device 10.

In any of the previously described embodiments of the testing device 10, the substrates of the testing device 10 that are arranged above the second substrate 14, for example the third substrate 16 in the embodiments illustrated in Fig. 1, 2 and 4, and also the fourth substrate 18 in the embodiment illustrated in Fig. 3, and also the fifth substrate 13 in the embodiment illustrated in Fig. 5, have a transparent portion that optical exposes the second substrate 14 to an exterior of the testing device from above, thereby allowing a user of the testing device 10 or a detection unit, possibly comprising an optical sensor, configured to detect a visual property to have visual access to the second substrate 14 from above. Thus, when the bacterial marking substance is configured to undergo a variation in a visual property, such as colouration, in the presence of bacteria, such property variations can be observed from the exterior of the testing device 10, either by the user or by said detection unit.

As part of a testing kit according to the invention, the testing device 10 according to any of the exemplary embodiments illustrated in any of Figs. 1 to 5 can comprise, in the respective substrates, a biological culture medium for supporting bacterial growth, a bacterial marking substance, and a sample of an antibiotic drug to be tested. The bacterial marking substance can be a chromogenic substance configured for undergoing a change in colouration in the presence of bacteria. The sample of the antibiotic drug can for example be a dry sample of an antibiotic drug to be tested embedded within the corresponding substrate in a localised proximity of the corresponding end substrate portion being or overlapping the end portion 14e of the second substrate 14.

When a biological probe of a patient, for example a urine probe, is added to the corresponding substrate, for example to the second substrate 14 or to the second partial substrate 14b, while the testing device 10 is in the open configuration illustrated in Fig. 1,3, 4, and 5, for example by exposing the tip (right end in Figs. 1, 3, 4, and 5) of second substrate 14 (or of the second partial substrate 14b) to a liquid containing the biological probe, the biological probe impregnates the second substrate 14 (or the second partial substrate 14b). When the testing device 10 is subsequently arranged in the closed configuration (cf. Fig. 2), the biological probe enters in contact with the remaining substances present in the second substrate 14 (or in the second partial substrate 14b) and in the adjacent substrates. Apart from mixing with the biological culture medium and with the chromogenic substance that possibly impregnate most of the second substrate 14, the biological probe can also reach and dilute the dry sample of the antibiotic drug, and hence also mix with the antibiotic drug.

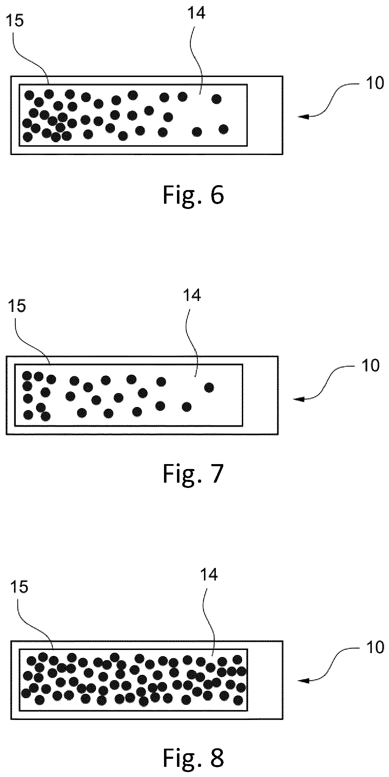

The reaction between the biological probe and the biological culture medium results in a reproduction of bacteria present in the biological probe, i.e. in bacterial growth. In the presence of such bacteria, the chromogenic substance undergoes a change in colouration that visually signals the presence of bacteria to a user of the testing device 10 through the transparent portions of the respective substrates. However, the reaction between the bacteria and the antibiotic drug can lead to a suppression of bacterial life and bacterial reproduction depending on whether the bacteria originally present in the biological probe are resistant or susceptible to the antibiotic drug. After a sufficient time of incubation during which the aforementioned reactions take place, for example after 24 or 48 hours, a user of the testing device 10 can obtain reliable information about the susceptibility of the bacteria present in the biological probe to the tested antibiotic drug. This information can be obtained in the form of a visual antibiogram, as illustrated in Fig. 6 to 8.

Fig. 6 to 8 schematically illustrate three different antibiograms that can be obtained with the testing device 10 of the invention in a top view after the incubation period, i.e. after the biological probe has reacted in the testing device 10 with the chromogenic substance and with the antibiotic drug, which may be a different drug for each of Fig. 6 to 8. The testing device 10 of each of Fig. 6 to 8 can correspond to any of the embodiments of the testing device 10 of the invention described above with respect to Fig. 1 to 5.

As seen in Figs. 6 to 8, the substrates of the testing device arranged between a topmost surface of the testing device 10 and the second substrate 14 (or the first partial substrate 14a in the case of the embodiment illustrated in Fig. 4) overlap with each other vertically forming a transparent window 15 of the testing device 10, through which the second substrate 14 is visible.

In Figs. 6 to 8, the density of the dots within the transparent window 15 is indicative of the intensity of a change in coloration undergone by the chromogenic substance after reacting with the biological probe in the presence of a sample of an antibiotic drug that was initially localized, as seen in Figs. 6 to 8, at a right end of the corresponding substrate.

If a localized suppression of bacterial growth is observed in the second substrate 14 around the initial location of the sample of an antibiotic drug in the second substrate 14 (i.e. if no change in the coloration of the chromogenic substance or only a reduced change is observed in said initial location), a certain degree of susceptibility can be inferred, wherein a greater degree of susceptibility will be signalled by a more intense and/or more extended colour suppression (cf. Fig. 6), whereas a lesser degree of susceptibility will be signalled by a less intense and/or less extended colour suppression (cf. Fig. 7). In this case, the antibiotic drug being tested can be considered as a valid candidate for treating a bacterial infection of the patient.

If no localized suppression of bacterial growth is observed in the second substrate 14 around the initial location of the sample of antibiotic drug in the second substrate 14, i.e. if an approximately homogeneous change in the colouration of the chromogenic substance is observed throughout the second substrate 14 despite the presence of the antibiotic drug (cf. Fig. 8), a resistance of the bacteria present in the biological probe to the antibiotic drug being tested must be assumed, for which the tested antibiotic drug should be discarded as an effective drug for treating the bacterial infection of the patient.

For example, in the light of the antibiograms illustrated in Figs. 6 to 8, it can be assumed that the drug tested in the example represented in Fig. 8 is not suitable for treating an infection caused by bacteria present in the biological probe and that the drugs tested in the examples represented in figs. 6 and 7 are suitable for treating the infection caused by bacteria present in the biological probe, with the drug tested in the example of Fig. 7 presumably being more effective than the drug tested in the example represented in Fig. 6.

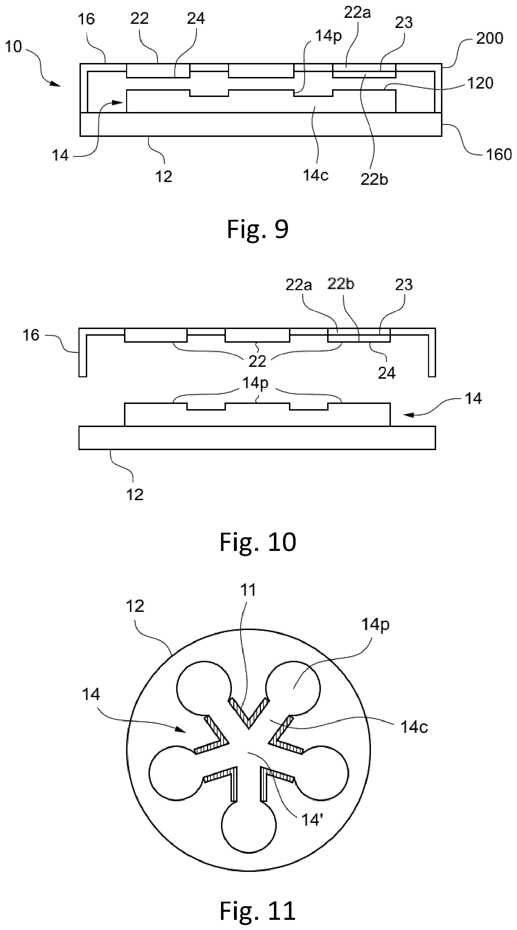

Figs. 9 to 12 show schematic views corresponding to a testing device 10 according to embodiments of the invention in which the testing device 10 has the previously described "multi-test configuration", allowing testing the susceptibility of a biological probe to a plurality of bioactive substances using one and the same testing device 10, possibly at the same time.

Fig. 9 shows a schematic side view of the testing device 10, in which the second substrate 14 is arranged on the first substrate 12 and comprises a plurality of probe receiving portions 14p. The test device 10 is a layer-based device having a layer structure, wherein the first substrate 12 is formed as a first substrate layer 160, the second substrate 14 is formed as a second substrate layer 120 stacked on the first substrate layer 160 and the third substrate 16 is formed as a third substrate layer 200 stacked on the second substrate layer 120. The testing device 10 can hence be manufactured layer-wise.

The first substrate 12 is made of polyethylene, the second substrate 14 is made of paper, and the third substrate 16 is made of transparent silicon or transparent plastic.

The second substrate 14, which is shown in more detail in a top view in Fig. 11, comprises a plurality of probe receiving portions 14p that are spatially separated from each other. In the example shown, the test device 10 is a circular-shaped portable test device and the second substrate 14 comprises five circular-shaped probe receiving portions 14p of equal sizes, but this is just an example and the testing devices 10 according to the invention may comprise any number of probe receiving portions 14p of circular shape or of other shapes. It should also be noticed, that in other embodiments, the testing device 10 and the substrates thereof need not be circular-shaped and may have a different shape, for example an oval shape, an elongated shape, polygonal shape or an irregular shape.

Fig. 11 shows a schematic top view of the testing device 10 of Figs. 9 and 10 with the third substrate 16 omitted for illustrative purposes. In the exemplary embodiment under consideration, the probe receiving portions 14p are symmetrically and regularly arranged on the circular-shaped first substrate 12, occupying positions that correspond to the vertices of a virtual regular pentagon on the first substrate 12. At the centre of said pentagon there is a common central section 14' of the second substrate 14, which is centrally arranged among the five probe receiving portions 14p and is fluidly connected to each one of them by a respective connection arm 14c of the second substrate 14. The probe receiving portions 14p and the respective connection arms 14c are separated from each other by a fluid-tight hydrophobic isolating element 11, which in the exemplary embodiment shown comprises a hydro-phobic wax.

The second substrate 14 is impregnated with a biological culture medium (e.g. lysogeny broth) for supporting bacterial proliferation and with a chromogenic substance, for example a pH sensitive dye, configured for undergoing a change in colouration when bacteria proliferate in the probe receiving portions 14p of the second substrate 14. In the exemplary embodiment under consideration, the probe receiving portions 14p are circular-shaped, the connection arms 13 are elongated in the radial direction(s) of the second substrate 14, and the biological culture and the chromogenic substance have homogeneous concentrations throughout the probe receiving portions 14p and the connection arms 14c of the second substrate 14.

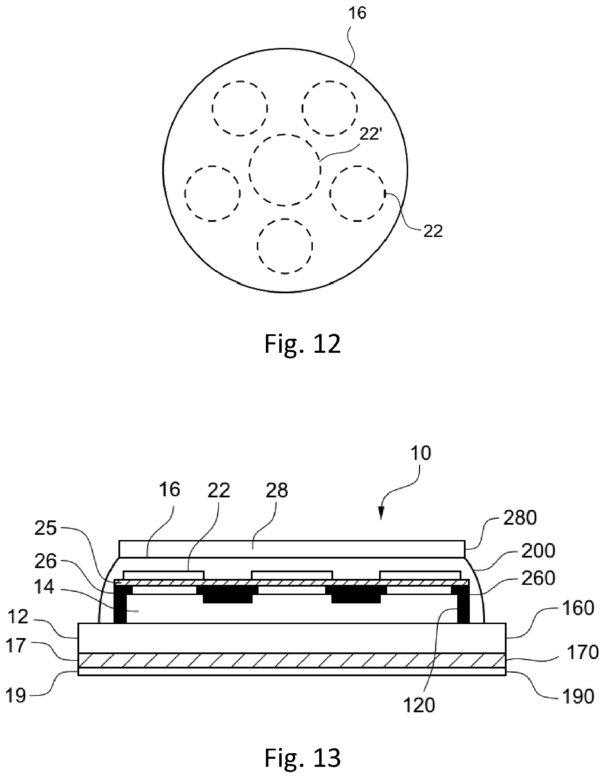

The third substrate 16, which is shown in more detail in a top view in Fig. 12, comprises a plurality of substance reservoirs 22 that are configured for receiving in their interior the one or more bioactive substances to be tested with the testing device 10. In particular, in the example under consideration, each of the substance reservoirs 22 may receive a different bioactive substance to be tested, such that, a testing kit according to the invention, which can comprise the testing device 10 with the second substrate 14 impregnated with the biological culture medium and with the chromogenic substance, and with the one or more bioactive substances contained in the substance reservoirs 22, allows testing the susceptibility of a biological probe to as many different drugs as substance reservoirs 22 the third substrate 16 has.

Each of the substance reservoirs 22 of the exemplary embodiments illustrated in Figs. 9 to 12 is releasably sealed by a corresponding individual releasable sealing element 24, which is formed as a breakable pressure-sensitive membrane, such that it is possible to easily unseal a given substance reservoir 22 by breaking the respective sealing element 24, for instance by applying pressure with the fingers.

The bioactive substances received in the substance reservoirs 22 may be provided in liquid, powder and/or solid form. As shown in Figs. 9 and 10, at least one of the substance reservoirs 22 can comprise a first cavity 22a and a second cavity 22b, wherein the first cavity 22a is releasably separated from the second cavity 22b by an intermediate sealing element 23, which may be similar in form and constitution to the sealing element 24. In such substance reservoirs 22, a bioactive substance may be provided in powder or solid form in one of the cavities, for example in the first cavity 22a, and a liquid solvent may be provided in the other cavity, for example in the second cavity 22b. Although the exemplary embodiment shown in Figs. 9 and 10 shows a single such substance reservoir having a first cavity 22a and a second cavity 22b, it is possible in other embodiments to have none of such substance reservoirs 22 having cavities, having all of the substance reservoirs 22 having such cavities or having a subgroup of the substance reservoirs 22 having such cavities and intermediate sealing elements 23.

As illustrated in Fig. 10, the third substrate 16 is movable with respect to the first substrate 12 and the second substrate 14. In the embodiment shown, the third substrate 16 is separable from first substrate 12 and the second substrate 14, although other configurations are possible. Fig. 9 illustrates a situation in which the testing device is in a closed configuration: the third substrate 16 is in a first position, in which the substance reservoirs 22 are arranged with each of the substance reservoirs 22 overlapping one of the probe receiving portions 14p of the second substrate 14 in a one-to-one spatial correspondence. Fig. 10 illustrates a situation in which the testing device 10 is in an open configuration: the third substrate 16 is in a second position, separated from the second substrate 14 and the first substrate 12, such that the probe receiving portions 14p of the second substrate 14 are exposed to an environment of the testing device 10 by the third substrate 16.

With the testing device 10 in the open configuration illustrated in Fig. 10, it is possible to dispense a biological probe, such as a urine probe, to the probe receiving portions 14p of the second substrate 14. For example, a patient or user of the testing device 10 may hold the second substrate 14, while exposed by the third substrate 16, in contact with a stream of urine in order to obtain a urine probe. It is also possible to dip the second substrate 14, while exposed by the third substrate 16, in a probe container containing the urine probe. The biological probe, in this case the urine probe, then impregnates the probe receiving portions 14p and the connection arms 14c of the second substrate 14, since the second substrate 14 is liquid-impregnable.

With the testing device 10 in the closed configuration illustrated in Fig. 9, it is possible to dispense the bioactive substances received in each of the substance reservoirs 22 to a corresponding underlying probe receiving portion 14p by simply unsealing the substance reservoirs 22, for example by breaking the corresponding sealing elements 24 that individually seal the substance reservoirs 22. Upon unsealing a substance reservoir 22, the bioactive substance contained therein is dispensed to the probe receiving portion 14p overlaid by the unsealed substance reservoir 22 and impregnates the second substrate 14 in an environment of the corresponding probe receiving portion 14p.

In the case of a substance reservoir 22 having a first cavity 22a and a second cavity 22b, the same action that removes or breaks the sealing element 24, for example the same braking pressure, or a similar action can also cause the intermediate sealing element 23 separating the first cavity 22a from the second cavity 22b to break, such that a powder-based or solid bioactive substance received in one of the cavities, for example in the first cavity 22a, dissolves into the liquid solvent received into the other cavity, for example into the second cavity 22b, and is then dispensed into the corresponding underlying probe receiving portion 14p as a liquid solution containing the bioactive substance, where it impregnates the second substrate 14 in an environment of the corresponding probe receiving portion 14p.

As shown in Fig. 12, the third substrate 16 further comprises, apart from the substance reservoirs 22, a disinfectant reservoir 22' containing a disinfectant such as ethanol. The disinfectant reservoir 22' is centrally arranged in the third substrate 16 among the substance reservoirs 22 and, in the closed configuration illustrated in Fig. 9, overlies the common central section 14' of the second substrate 14. The disinfectant reservoir is sealed by a corresponding sealing element (not shown) and can be selectively unsealed in the same manner as any other reservoir. In particular, the disinfectant reservoir can be left unsealed while performing the susceptibility test with the testing device 10 and once the test results are obtained, the disinfectant reservoir 22' can be unsealed - while having the testing device 10 in the closed position shown in Fig. 1 - thereby causing the disinfectant to impregnate the second substrate 14 extending outwardly from the common central section 12', such that the testing device 10 is sterilized.

Figs. 13 to 15 show schematic views corresponding to a further testing device 10 according to embodiments of the invention.

The testing device 10 of Figs. 13 to 15 comprises the same components as the testing device described with respect to Figs. 9 and 10, in particular the first substrate 12, formed as a first substrate layer 160, the second substrate 14, formed as a second substrate layer 120 on the first substrate layer 160, and the third substrate 16, formed as a third substrate layer 200 above the second substrate layer 120 and the first substrate layer 160.

The third substrate 16 of the testing device 10 of Figs. 13 to 15 differs from the third substrate 16 of the testing device of Figs. 9 and 10 by having a common releasable sealing element 25 that releasably seals all substance reservoirs 22 (instead of the "individual" sealing elements 24). The substance reservoirs 22 can hence be unsealed at once by removing the releasable sealing element 25. The releasable sealing element 25 can be formed as a removable sealing membrane made. In embodiments having a disinfectant reservoir 22' (cf. Fig. 12), it is possible for the removable sealing element 25 to have an annular shape exposing a breakable sealing element (not shown) that releasably seals a disinfectant reservoir 22', such that the substance reservoirs 22 and the disinfectant reservoir can be selectively unsealed independently from each other.

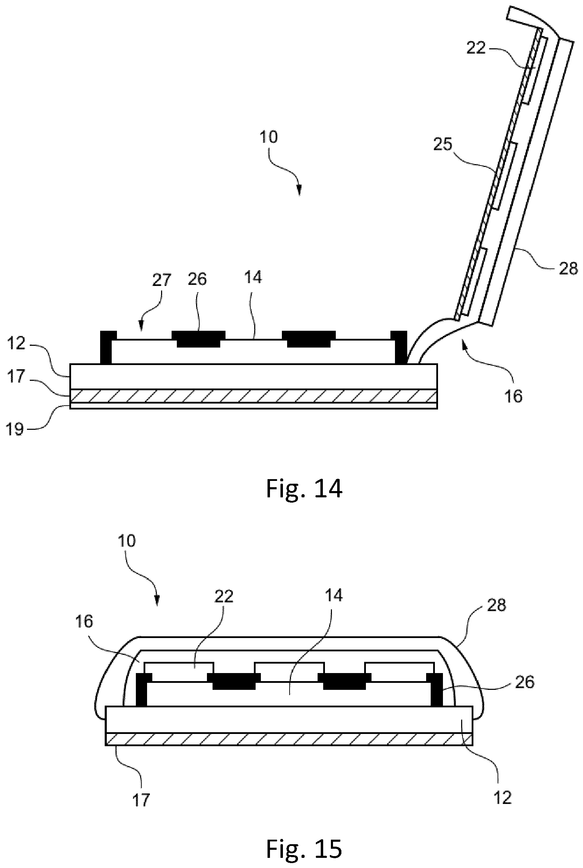

A further difference between the third substrate 16 of the testing device 10 of Figs. 13 to 15 with respect to the third substrate 16 of the testing device described with respect to Figs. 9 to 12 is that, in the exemplary embodiment shown in Figs. 13 to 15, as shown in particular in Fig. 14, the third substrate 16 is not completely separable from the first substrate 12 and the second substrate 14. Instead, the third substrate 16 is flexibly and pivotably attached to the first substrate 12, such that it can be moved between a closed configuration (see Fig. 13) and an open configuration (see Fig. 14) - corresponding to the respective configurations previously described for the testing device of Figs. 1 to 5 and for the testing device of Figs. 9 to 12 - by pivoting the third substrate 16 without completely detaching it from the rest of the testing device 10.

A further difference between the third substrate 16 of the testing device 10 of Figs. 13 to 15 with respect to the third substrate 16 of the testing device described with respect to Figs. 9 to 12 is that, in the exemplary embodiment shown in Figs. 13 to 15, some of the substance reservoirs 22 contain, instead of a bioactive substance as such, an antimicrobial disc (not shown) impregnated with the bioactive substance to be tested. When the substance reservoirs 22 are released, the corresponding antimicrobial discs enter into contact with the respective underlying probe receiving portion 14p, such that the bioactive substance can react with the biological probe impregnating said probe receiving portion 14p.

The testing device 10 of Figs. 13 to 15 further includes a biocompatible adhesive 17, for example a tackified acrylic adhesive, formed as an adhesive layer 170 that is attached to a surface of the first substrate 12 opposite to the second substrate 14. The adhesive 17 is suitable for attaching the testing device 10 to the body, e.g. to the skin, of a human or animal patient. The adhesive 17 is removably covered by a removable cover 19, which is formed as a removable cover layer 190 and which may for example be a peelable plastic sheet.

The testing device 10 of Figs. 13 to 15 further comprises a separation sheet 26 that is arranged between the third substrate 16, the second substrate 14 and the first substrate 12, extending between the probe receiving portions 14p of the second substrate 14. The separation sheet 26 is formed as a separation sheet layer 260. The separation sheet 26 can be made of a plastic material and comprises, for each of the probe receiving portions 14p (and for the central probe receiving portion 14' shown in Fig. 11) of the second substrate 14, a corresponding opening 27 exposing the respective probe receiving portion 14, 14'. The separation sheet 26 protects the probe receiving portions of the second substrate 14 from being damaged during manipulation of the testing device 10.

The testing device 10 of Figs. 13 to 15 further comprises a protective foil 28, which is formed as a protective foil layer 280 configured as a fifth substrate overlying the third substrate 16. The protective foil 28 is made of a radiation-shielding, light-shielding, deformable and thermally insulating material. The protective foil 28 is configured for covering the third substrate 16 for providing radiation-shielding protection to the substances contained within the reservoirs 22 and, once used to wrap the testing device 10, in particular for incubation or after obtaining the susceptibility results, for providing air-tight and fluid-tight protection to the interior of the testing device 10 in order to avoid contamination or leakage to the environment.

Fig. 15 shows a configuration in which the testing device 10 of Figs. 13 and 14 has been returned to the closed configuration, wherein the removable cover 19 and the releasable sealing element 25 have been removed and wherein the elastic protective foil 28 has been used to wrap the testing device 10 in a fluid-tight manner.

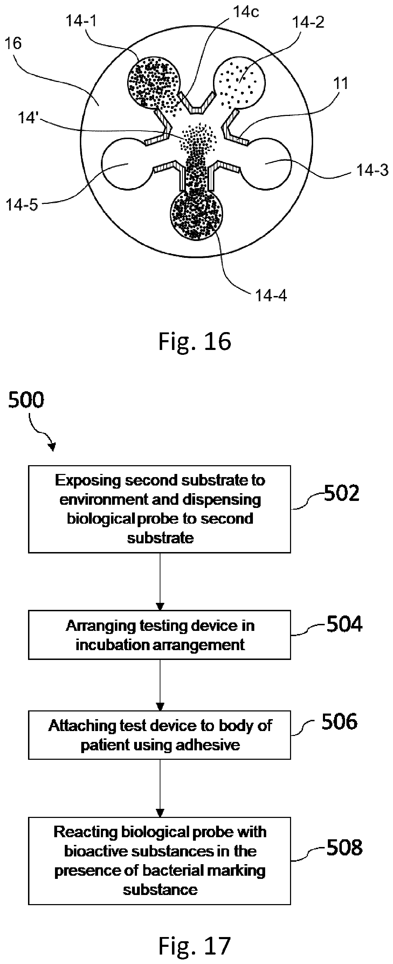

Fig. 16 illustrates a schematic top view of the testing device 10 of Figs. 9 to 12 or 13 to 15, wherein the third substrate 16 is omitted for illustrative purposes, although the visual result visible in Fig. 16 can be visible from a real top view also with the third substrate 16, in particular through a transparent portion of the third substrate 16. The third substrate 16 can also be made of a transparent material.

As shown in Fig. 16, the probe receiving portions 14-1 to 14-5 of the second substrate 14 have the same arrangement as the probe receiving portions 14p described with respect to the embodiment illustrated in Fig. 11, the only difference being the presence of a central dummy probe receiving portion 14' of the second substrate 14 being fluidly connected to each of the remaining probe receiving portions 14-1 to 14-5 by a respective connection arm 14c. The dummy probe receiving portion 14' is hence formed as a central probe receiving portion arranged among the remaining probe receiving portions 14-1 to 14-5.

The reservoir of the third substrate 16 that is arrangeable directly above the dummy probe receiving portion 14' at the centre of the testing device 10 needs not contain any bioactive substance and can instead be a disinfectant reservoir 22' containing a disinfectant, like for example ethanol.

Fig. 16 shows an exemplary result that may be obtained with a testing device 10 according to the "multi-test configuration" of the invention when testing the susceptibility of five different bioactive substances respectively dispensed into the probe receiving portions 14-1 to 14-5. The density of the dotted regions indicate the intensity of a change of colouration of the chromogenic substance that impregnates the second substrate 14, which is also impregnated with a biological culture medium. All probe receiving portions 14-1 to 14-5 of the second substrate 14 have the same an homogenous concentration of the chromogenic substance, such that the extension, density and/or intensity of the change in colouration registered for the chromogenic substance in the different probe receiving portions 14-1 to 14-5 and in the respective connection arms 14c is indicative of the quantity of proliferating bacteria. The results obtained for each of the different drugs can be directly compared to each other, since the same biological probe is dispensed to all probe receiving portions 14-1 to 14-5.

The change of colouration in the central/dummy probe receiving portion 14', which is not impregnated with any bioactive substance and hence acts as a negative control, confirms the presence of bacteria in the biological probe that has been dispensed to the probe receiving portions of the second substrate 14. The change of colouration undergone by the chromogenic substance in each of the different probe receiving portions 14-1 to 14-5 and the corresponding connection arms 14c is indicative of the susceptibility of the bacteria present in the biological probe to the each of the different bioactive substances being tested. For the bioactive substances dispensed into the probe receiving portions 14-1 and 14-4, the biological probe shows a high degree of immunity to the corresponding bioactive substance. For the bioactive substance dispensed into the probe receiving portion 14-2 the biological probe shows a high degree of susceptibility, and for the bioactive substances dispensed into the probe receiving portions 14-3 and 14-5, the biological probe presents no immunity at all, i.e. has a high degree of susceptibility, for bacterial growth is suppressed to a very high degree. As a result, the bioactive substances administered into the probe receiving portions 14-3 and 14-5 can be identified as potentially effective for suppressing bacteria in the biological probe, whereas the bioactive substance administered into the other probe receiving portions 14-1, 14-2 and 14-4 can be discarded for that purpose.

Once the results of the susceptibility test have been obtained with a testing device 10, the biological probe present in the probe receiving portions of the second substrate 14 can be sterilized using the disinfectant contained in the disinfectant reservoir 22' by unsealing the disinfectant reservoir 22', for example by breaking the sealing element 24 of the disinfectant reservoir 22' by applying a mechanical pressure. The disinfectant, which can for example be pure ethanol, is then dispensed to the central probe receiving portion 14' and impregnates from there the entire second substrate 14, sterilizing it.

It is also possible, however, for example in the exemplary embodiment illustrated in Fig. 9 to 12, for the disinfectant to be dispensed into a volume that is in fluid communication with the probe receiving portions 14p (eg. intermediate volume between the second substrate 14 and the third substrate 16 in Fig. 1). The quantity of disinfectant can be such as to completely fill such intermediate volume, thereby sterilizing the probe receiving portions of the second substrate 14.

Fig. 17 is a flow diagram schematically illustrating a method 500 of testing the susceptibility of the biological probe to one or more bioactive substances using a testing device 10 or a testing kit according to the invention comprising such a testing device 10. The method 500 can be performed using any of the testing devices 10 described above with respect to Figs. 1 to 15.

In step 502, the method comprises exposing at least a portion of the second substrate 14 of the testing device 10 to biological probe obtained from a flow of liquid comprising a biological probe or from a probe recipient containing such liquid. For example, the at least one portion of the second substrate 14 of the testing device 10 can be directly exposed to a flow of liquid comprising the biological probe or can be soaked with said liquid by dipping the at least a portion of the second substrate 14 of the testing device into the probe recipient containing the liquid. However, it is also possible to dispense the biological probe into the at least one portion of the second substrate 14 using another substrate arrangeable in contact with the second substrate 14. During step 502, the testing device 10 can be in the open configuration described above with respect to the exemplary embodiments shown in Figs. 1, 3, 4, 5, 10 and 14.

In step 504, the testing device 10 is arranged in the incubation arrangement, which can correspond to the arrangement described above with respect to the exemplary embodiments shown in Figs. 2, 9 and 15. In this arrangement, the second substrate 14 is protected from an environment of the testing device 10 by the remaining substrate of the plurality of substrates of the testing device 10.

In step 506, the testing device 10 is attached to the body of a patient using an adhesive arranged on the first substrate 12 of the testing device 10 for incubation using the body warmth of the patient.

In step 508, during incubation, the biological probe is made to react with one or more bioactive substances in the testing device 10 in the presence of a bacterial marking substance that is configured for undergoing a property change in the presence of bacteria.

By means of the method 500, susceptibility results like the exemplary susceptibility results illustrated and described above in Figs. 6 to 8 and 16 can be obtained.

Although preferred exemplary embodiments are shown and specified in detail in the drawings and the preceding specification, these should be viewed as purely exemplary and not as limiting the invention. It is noted in this regard that only the preferred exemplary embodiments are shown and specified, and all variations and modifications should be protected that presently or in the future lie within the scope of protection of the invention as defined in the claims.

## Claims

1. A testing device (10) for testing the susceptibility of a biological probe to one or more bioactive substances, wherein the device comprises a plurality of substrates comprising:
a first substrate (12), wherein the first substrate is liquid-impermeable,
a second substrate (14) arranged or arrangeable above the first substrate (12), wherein the second substrate (14) is liquid-permeable and liquid-impregnable, and
a third substrate (16) arranged or arrangeable above the second substrate (14), wherein the third substrate (16) is liquid-impermeable;
wherein the first substrate and/or the third substrate are configured for encapsulating the second substrate,
wherein at least a portion of the second substrate (14) is exposable to an environment of the testing device for receiving the biological probe.

2. The testing device (10) of claim 1, wherein the second substrate (14) comprises or is made of a liquid-permeable material, wherein the liquid-permeable material preferably is a porous material, in particular paper, preferably having a pore size of 0.3 µm or less, more preferably of 0.1 µm or less.

3. The testing device (10) of claim 1 or 2, wherein the first substrate (12) comprises an adhesive material, in particular an adhesive layer (17), for adhering the testing device to a human or animal body, and wherein the testing device preferably further comprises a removable cover (19) covering the adhesive material.

4. The testing device (10) of any of the preceding claims, wherein the substrates of the plurality of substrates arranged above the second substrate (14) are at least partly transparent, such as to allow an optical access to the second substrate (14) from an exterior of the testing device.

5. The testing device (10) of any of the preceding claims, wherein at least some of the substrates of the plurality of substrates are formed as respective substrate layers.

6. The testing device (10) of any of the preceding claims, wherein the second substrate (14) is arrangeable in a probe collection arrangement and in an incubation arrangement, wherein in the probe collection arrangement, the at least one portion of the second substrate (14) is exposed to an environment of the testing device for receiving the biological probe, and wherein in the incubation arrangement, the second substrate (14) is protected from said environment of the testing device, in particular by the remaining substrates of the plurality of substrates; and/or
wherein at least some of the substrates of the plurality of substrates are mutually attached or attachable by respective mutually overlapping attachment portions, preferably corresponding to respective end portions (12e, 14e, 16e) of the substrates of the plurality of substrates, wherein in an open configuration of the plurality of substrates, at least some of the substrates of the plurality of substrates are spread apart from each other, such that the at least one portion of the second substrate (14) is exposed to an environment of the testing device for receiving the biological probe, and wherein in a closed configuration of the plurality of substrates, the substrates of the plurality of substrates completely overlap each other.

7. The testing device (10) of any of the preceding claims, further comprising at least one substance pad (9) configured for receiving a respective one of the one or more bioactive substances, wherein the at least one substance pad (9) is in particular arranged on one of the substrates of the plurality of substrates other than the second substrate (14) adjacent to the second substrate (14), such that the at least one substance pad (9) faces the second substrate (14), wherein the at least one substance pad (9) preferably comprises at least one antimicrobial susceptibility disc.

8. The testing device (10) of any of the preceding claims, further comprising a fourth substrate (18) arranged or arrangeable between the second substrate (14) and the first substrate (12) or the third substrate (16), wherein the fourth substrate (18) is liquid-impregnable.

9. The testing device (10) of any of the preceding claims, wherein the second substrate (14) comprises a first partial substrate (14a) and a second partial substrate (14b) separable from the first partial substrate (14a), wherein the first partial substrate (14a) preferably is liquid-permeable, and wherein the second partial substrate (14b) preferably is liquid-impregnable and corresponds to the at least one portion of the second substrate (14) exposable to an environment of the testing device for receiving the biological probe.

10. The testing device (10) of any of the preceding claims, wherein the plurality of substrates further comprises a fifth substrate (13) arranged or arrangeable above the remaining substrates of the plurality of substrates, in particular above the third substrate (16), wherein the fifth substrate (13) is radiation shielding, light shielding, thermally insulating and/or air-tight.

11. The testing device (10) of any of the preceding claims, further comprising a disinfectant reservoir (7) configured for receiving a substance comprising a disinfectant, wherein the disinfectant reservoir (7) is releasably sealable or sealed by a corresponding sealing element, and wherein the disinfectant reservoir is preferably arranged facing the second substrate (14).

12. The testing device (10) of any of the preceding claims, wherein the second substrate (14) comprises one or more probe receiving portions (14p) spatially separated from each other;
wherein the third substrate (16) comprises one or more substance reservoirs (22), wherein each of the one or more substance reservoirs (22) is configured for receiving one of the one or more bioactive substances and is releasably sealed by a sealing element (24);
wherein each of the one or more substance reservoirs (22) is arranged or arrangeable overlapping at least one of the probe receiving portions (14p), such that, when one of the substance reservoirs (22) is unsealed, the corresponding bioactive substance is released into a corresponding probe receiving portion (14p) overlapped by said substance reservoir (22), and
wherein the one or more probe receiving portions (14p) are exposed or exposable to the environment of the testing device for receiving the biological probe;
wherein the third substrate (16) is preferably movable with respect to the first substrate (12) and/or the second substrate (14), wherein preferably, in a first position of the third substrate (16), each of the one or more substance reservoirs (22) is arranged overlapping at least one of the probe receiving portions (14p), and wherein in a second position of the third substrate (16), the one or more probe receiving portions (14p) are exposed to the environment of the testing device (10); and/or
wherein at least a part of the probe receiving portions (14p) are preferably arranged regularly and/or symmetrically with respect to each other, in particular on the first substrate (12), more preferably around a common central section (14') of the second substrate (14) and connected therewith by respective connection arms (14c) of the second substrate (14).

13. A testing kit for testing the susceptibility of a biological probe to one or more bioactive substances, wherein the testing kit comprises a testing device (10) according to any of the preceding claims and at least one of: one or more bioactive substances, a biological culture medium, and a bacterial marking substance configured for undergoing a property change in the presence of bacteria;
wherein the bacterial marking substance preferably is or comprises a chromogenic substance configured for undergoing a change of colouration in the presence of bacteria; and/or
wherein some or all of the one or more bioactive substances preferably comprise or are antibiotic drugs.

14. The testing kit of claim 13, further comprising a probe recipient configured for receiving a liquid containing the biological probe, wherein the at least one portion of the second substrate (14) of the testing device (10) is insertable into the probe recipient, in particular when the biological probe is received in the probe recipient, for being impregnated with the biological probe.

15. A method (500) of testing the susceptibility of a biological probe to one or more bioactive substances using a testing device (10) or a testing kit according to any of the preceding claims, wherein the method comprises:
dispensing (520) the biological probe into at least a portion of the second substrate (14) of the testing device (10); and
reacting the biological probe with the one or more bioactive substances in the presence of a bacterial marking substance configured for undergoing a property change in the presence of bacteria;
wherein dispensing (520) the biological probe into at least a portion of the second substrate (14) preferably comprises exposing (510) the at least one portion of the second substrate (14) to a flow of a liquid comprising the biological probe or soaking the at least one portion of the second substrate (14) with said liquid, more preferably with said liquid being received in a probe recipient; and/or
wherein the method preferably further comprises, after dispensing (520) the biological probe into the at least one portion of the second substrate (14) of the testing device (10) and before and/or while reacting the biological probe with the one or more bioactive substances, arranging the testing device (10) in an incubation arrangement, in which the second substrate (14) is protected from an environment of the testing device (10), in particular by the remaining substrates of the plurality of substrates.
